# EUROPEAN PATENT SPECIFICATION

(11) **EP 3 823 972 B1**
(45) Date of publication and mention of the grant of the patent: **07.09.2022**
(21) Application number: 19745231.1
(22) Date of filing: 19.07.2019
(51) Int. Cl.: C07D 487/04, A61K 31/4985, A61P 17/00

(54) **IMIDAZO[1,2-B]PYRIDAZINE DERIVATIVES AS TRK INHIBITORS**
IMIDAZO[1,2-B]PYRIDAZINE VERBINDUNGEN ALS TRK INHIBITOREN
DÉRIVÉS DE IMIDAZO[1,2-B]PYRIDAZINE EN TANT QU' INHIBITEURS TRK

(30) Priority: 19.07.2018 GB 201811825
(43) Date of publication of application: 26.05.2021
(73) Proprietor: BenevolentAI Bio Limited, London W1T 5HD (GB)
(72) Inventor: BROWN, Alan, London W1T 5HD (GB); GLEN, Angela, London W1T 5HD (GB)
(74) Representative: Gill Jennings & Every LLP
(86) International application number: PCT/GB2019/052021
(87) International publication number: WO 2020/016594

(56) References cited:
- WO-A1-2012/034091
- US-A1- 2006 025 614

## Description

The present invention relates to certain imidazo[1,2-b]pyridazine compounds and the pharmaceutically acceptable salts of such compounds, compositions containing the compounds, and the uses of such compounds and salts in treating diseases or conditions associated with tropomyosin-related kinase (Trk), activity. More specifically the invention relates to the compounds and their salts useful as inhibitors of Trk. Tropomyosin-related kinases (Trks) are a family of receptor tyrosine kinases activated by neurotrophins, a group of soluble growth factors including Nerve Growth Factor (NGF), Brain-Derived Neurotrophic Factor (BDNF) and Neurotrophin-3 (NT-3) and Neurotrophin-4/5 (NT-4/5). The Trk receptors include three family members TrkA, TrkB and TrkC that bind to and mediate the signal transduction derived from the Neurotrophins. NGF activates TrkA, BDNF and NT-4/5 activate TrkB and NT3 activates TrkC.

Tropomyosin-related kinases have been implicated in the following diseases: atopic dermatitis, psoriasis, eczema and prurigo nodularis, acute and chronic itch, pruritus, inflammation, cancer, restenosis, atherosclerosis, thrombosis, pruritus, lower urinary tract disorder, inflammatory lung diseases such as asthma, allergic rhinitis, lung cancer, psoriatic arthritis, rheumatoid arthritis, inflammatory bowel diseases such as ulcerative colitis, Crohn's disease, fibrosis, neurodegenerative disease, diseases disorders and conditions related to dysmyelination or demyelination, certain infectious diseases such as *Trypanosoma cruzi* infection, (Chagas disease), cancer related pain, chronic pain, neuroblastoma, ovarian cancer, colorectal cancer, melanoma, head and neck cancer, gastric carcimoma, lung carcinoma, breast cancer, glioblastoma, medulloblastoma, secratory breast cancer, salivary gland cancer, papillary thyroid carcinoma, adult myeloid leukaemia, tumour growth and metastasis and interstitial cystitis. (C. Potenzieri and B. J. Undem, Clinical & Experimental Allergy, 2012 (42) 8-19; Yamaguchi J, Aihara M, Kobayashi Y, Kambara T, Ikezawa Z, J Dermatol Sci. 2009;53:48-54; Dou YC, Hagstromer L, Emtestam L, Johansson O., Arch Dermatol Res. 2006;298:31-37; Johansson O, Liang Y, Emtestam L., Arch Dermatol Res. 2002;293:614-619; Grewe M, Vogelsang K, Ruzicka T, Stege H, Krutmann J., J Invest Dermatol. 2000;114:1108-1112; Urashima R, Mihara M .Virchows Arch. 1998;432:363-370; Kinkelin I, Motzing S, Koltenzenburg M, Brocker EB., Cell Tissue Res. 2000;302:31-37; Tong Liu & Ru-Rong Ji, Pflugers Arch - Eur J Physiol, DOI 10.1007/s00424-013-1284-2, published online 1 May 2013.); International Patent Application publication numbers WO2012/158413, WO2013/088256, WO2013/088257 and WO2013/161919, (Brodeur, G. M., Nat. Rev. Cancer 2003, 3, 203-216), (Davidson. B. , et al. , Clin. Cancer Res. 2003, 9, 2248-2259), (Bardelli, A , Science 2003, 300, 949), (Truzzi, F. , et al. , Dermato-Endocrinology 2008, 3 (I), pp. 32-36), Yilmaz,T. , et al. , Cancer Biology and Therapy 2010, 10 (6), pp. 644-653), (Du, J. et al. ,World Journal of Gastroenterology 2003, 9 (7), pp. 1431-1434), (Ricci A, et al., American Journal of Respiratory Cell and Molecular Biology 25 (4), pp. 439-446), (Jin, W. , et al. , Carcinogenesis 2010, 31 (11), pp. 1939-1947), (Wadhwa, S. , et al., Journal of Biosciences 2003, 28 (2), pp. 181-188), (Gruber-Olipitz, M. , et al. , Journal of Proteome Research 2008, 7 (5), pp. 1932-1944), (Euthus, D. M. et al. , Cancer Cell 2002, 2 (5), pp. 347-348),(Li, Y. -G. , et al., Chinese Journal of Cancer Prevention and Treatment 2009, 16 (6), pp. 428-430), (Greco, A , et al. , Molecular and Cellular Endocrinology 2010, 321 (I), pp. 44-49), (Eguchi, M., et al., Blood 1999, 93 (4), pp. 1355-1363), (Nakagawara, A (2001) Cancer Letters 169: 107-114; Meyer, J. et al. (2007) Leukemia,1-10; Pierottia, M. A and Greco A, (2006) Cancer Letters 232:90- 98; Eric Adriaenssens, E., et al. Cancer Res (2008) 68:(2) 346-351 ), (FreundMichel, V; Frossard, N. , Pharmacology ck Therapeutics (2008) 117(1), 52-76), (Hu Vivian Y; et. al. The Journal of Urology (2005), 173(3), 1016-21), (Di Mola, F. F, et. al. Gut (2000) 46(5), 670-678) (Dou, Y. -C. ,et. al. Archives of Dermatological Research (2006) 298(1), 31-37), (Raychaudhuri, S. P. , et al. , J. Investigative Dermatology (2004) 122(3), 812-819) and (de Melo-Jorge, M. et al. , Cell Host ck Microbe (2007) 1 (4), 251-261).

WO 2012/034091 relates to imidazo[1,2]pyridazine compounds and their use in treating or preventing diseases or conditions associated with abnormal or deregulated TRK kinase activity.

US 2006/025614 relates to cyanoamidines as antagonists of the P2X7 receptor. Such compounds may be useful in the treatment of pain, imflammation and neurodegeneration.

In the following, reference will be made to subject-matter/embodiments which are part of the disclosure but not falling under the intended scope of protection, which is solely defined by the appended claims.

In one aspect, the invention provides a compound of Formula (I): 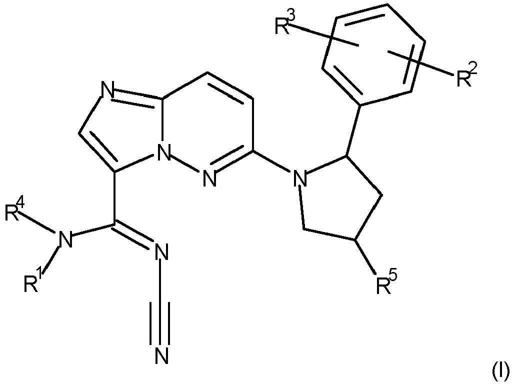 or a pharmaceutically acceptable salt or solvate thereof;
wherein:
R¹ is selected from H, -XR⁷, (C₁-C₆)alkyl, (C₃-C₈)cycloalkyl, and a C-linked 4-6 membered heterocycloalkyl containing 1 to 2 heteroatoms selected from N, O and S;
X is selected from -CH₂-;
R² is selected from H and -SR⁶;
R³ is selected from H and halo;
R⁴ is selected from H and (C₁-C₃)alkyl
R⁵ is selected from H and halo;
R⁶ is methyl;
R⁷ is phenyl substituted by hydroxy wherein the hydroxyphenyl is optionally further substituted by halo;
provided that if R² is H then R¹ is XR⁷.

In one embodiment of the invention as defined anywhere above, R¹ is selected from -XR⁷, (C₁-C₆)alkyl, (C₃-C₈)cycloalkyl, and a C-linked 4-6 membered heterocycloalkyl containing 1 to 2 heteroatoms selected from N, O and S.

In a further embodiment of the invention as defined anywhere above, R¹ is selected from (C₁-C₆)alkyl and (C₃-C₈)cycloalkyl

In an alternative further embodiment of the invention as defined anywhere above, R¹ is selected from -XR' and a C-linked 4-6 membered heterocycloalkyl containing 1 to 2 heteroatoms selected from N, O and S.

In a yet further embodiment of the invention as defined anywhere above, R¹ is (C₁-C₆)alkyl

In a yet further embodiment of the invention as defined anywhere above, R¹ is selected from -XR⁷ and a C-linked 4-6 membered heterocycloalkyl containing 1 to 2 heteroatoms selected from N and O.

In another embodiment of the invention as defined anywhere above, R² is -SR⁶.

In another embodiment of the invention as defined anywhere above, R³ is H or fluoro.

In another embodiment of the invention as defined anywhere above, R⁴ is H.

In another embodiment of the invention as defined anywhere above, R⁵ is H or fluoro.

In another embodiment of the invention as defined anywhere above, R⁷ is phenyl substituted by hydroxy wherein the hydroxyphenyl is optionally further substituted by fluoro.

In a yet further embodiment, the invention provides a compound of Formula Ia 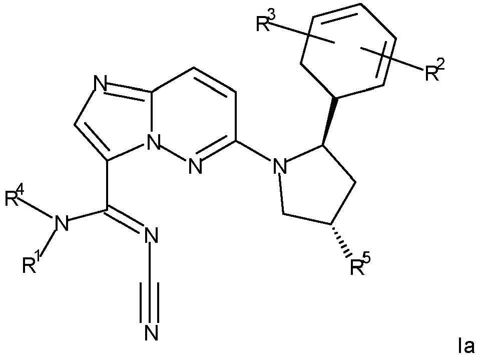 or a pharmaceutically acceptable salt or solvate thereof wherein R¹, R², R³, R⁴and R⁵ are as defined anywhere hereinabove in respect of a compound of Formula I.

In another embodiment, individual compounds according to the invention are those listed in the Examples section below.

In another embodiment of the invention, there is provided a compound according to the invention which is selected from Examples 1, 2, 3, 4, 5, 6 and 7 or a pharmaceutically acceptable salt or solvate thereof.

In another embodiment of the invention, there is provided a compound according to the invention which is selected from:
N'-cyano-6-[2-[5-fluoro-2-(methylsulfanyl)phenyl]pyrrolidin-1-yl]-N-[(3R)-oxan-3-yl]imidazo[1,2-b]pyridazine-3-carboximidamide;
N'-cyano-6-[2-[5-fluoro-2-(methylsulfanyl)phenyl]pyrrolidin-1-yl]-N-[(oxan-3-yl]imidazo[1,2-b]pyridazine-3-carboximidamide;
N'-cyano-N-ethyl-6-[4-fluoro-2-[5-fluoro-3-(methylsulfanyl)phenyl]pyrrolidin-1-yl]imidazo[1,2-b]pyridazine-3-carboximidamide;
N'-cyano-N-ethyl-6-[4-fluoro-2-[5-fluoro-2-(methylsulfanyl)phenyl]pyrrolidin-1-yl]imidazo[1,2-b]pyridazine-3-carboximidamide;
N-butyl-N'-cyano-6-[4-fluoro-2-[5-fluoro-2-(methylsulfanyl)phenyl]pyrrolidin-1-yl]imidazo[1,2-b]pyridazine-3-carboximidamide;
N'-cyano-N-cyclohexyl-6-[4-fluoro-2-[5-fluoro-2-(methylsulfanyl)phenyl]pyrrolidin-1-yl]imidazo[1,2-b]pyridazine-3-carboximidamide; and
N'-cyano-6-[2-[5-fluoro-2-(methylsulfanyl)phenyl]pyrrolidin-1-yl]-N-[(3-hydroxyphenyl)methyl]imidazo[1,2-b]pyridazine-3-carboximidamide;
or a pharmaceutically acceptable salt or solvate thereof.

In another embodiment of the invention, there is provided a compound according to the invention which is selected from:
(Z)-N'-cyano-6-[(2R)-2-[5-fluoro-2-(methylsulfanyl)phenyl]pyrrolidin-1-yl]-N-[(3R)-oxan-3-yl]imidazo[1,2-b]pyridazine-3-carboximidamide;
(Z)-N'-cyano-6-[(2R)-2-[5-fluoro-2-(methylsulfanyl)phenyl]pyrrolidin-1-yl]-N-[(3S)-oxan-3-yl]imidazo[1,2-b]pyridazine-3-carboximidamide;
(Z)-N'-cyano-N-ethyl-6-[(2R,4S)-4-fluoro-2-[5-fluoro-3-(methylsulfanyl)phenyl]pyrrolidin-1-yl]imidazo[1,2-b]pyridazine-3-carboximidamide;
(Z)-N'-cyano-N-ethyl-6-[(2R,4S)-4-fluoro-2-[5-fluoro-2-(methylsulfanyl)phenyl]pyrrolidin-1-yl]imidazo[1,2-b]pyridazine-3-carboximidamide;
(Z)-N-butyl-N'-cyano-6-[(2R,4S)-4-fluoro-2-[5-fluoro-2-(methylsulfanyl)phenyl]pyrrolidin-1-yl]imidazo[1,2-b]pyridazine-3-carboximidamide;
(Z)-N'-cyano-N-cyclohexyl-6-[(2R,4S)-4-fluoro-2-[5-fluoro-2-(methylsulfanyl)phenyl]pyrrolidin-1-yl]imidazo[1,2-b]pyridazine-3-carboximidamide; and
(Z)-N'-cyano-6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboximidamide;
or a pharmaceutically acceptable salt or solvate thereof.

In the embodiments mentioned herein, where only certain variables are defined, it is intended that the remainder of the variables are as defined in any embodiment herein. Thus, the invention provides for the combination of limited or optional definitions of variables.

The following terms as used herein are intended to have the following meanings: "Optionally substituted" as used herein means the group referred to can be unsubstituted, or substituted at one or two or three positions by any one or any combination of the substituents listed thereafter.

As used herein, the term "halogen" or "halo" refers to fluoro, chloro, bromo, and iodo. As used herein, the term "alkyl" refers to a fully saturated branched or unbranched hydrocarbon moiety having up to 20 carbon atoms. Unless otherwise provided, alkyl refers to hydrocarbon moieties having 1 to 16 carbon atoms, 1 to 10 carbon atoms, 1 to 7 carbon atoms, or 1 to 4 carbon atoms. Representative examples of alkyl include methyl, ethyl, *n*-propyl, *iso*-propyl, *n*-butyl, *sec*-butyl, *iso*-butyl, *tert*-butyl, *n-*pentyl, isopentyl, neopentyl, *n*-hexyl, 3-methylhexyl, 2,2- dimethylpentyl, 2,3-dimethylpentyl, *n*-heptyl, *n*-octyl, *n*-nonyl, *n*-decyl.

"C₁-C₃ alkyl", "C₁-C₆ alkyl", "C₁-C₈ alkyl" as used herein, denotes an alky group that contains one to three, six or eight (or the relevant number) carbon atoms. As used herein, the term "cycloalkyl" refers to saturated or unsaturated non-aromatic monocyclic, bicyclic or tricyclic hydrocarbon groups of 3-12 carbon atoms. Unless otherwise provided, cycloalkyl refers to cyclic hydrocarbon groups having between 3 and 9 ring carbon atoms or between 3 and 7 ring carbon atoms. Exemplary monocyclic hydrocarbon groups include, cyclopropyl, cyclobutyl, cyclopentyl cyclopentenyl, cyclohexyl and cyclohexenyl.

Exemplary bicyclic hydrocarbon groups include bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl "C₃-C₈-cycloalkyl" denotes a cycloalkyl group having 3 to 8 ring carbon atoms, for example a monocyclic group such as a cyclopropyl, cyclobutyl, cyclopentyl or cyclohexyl, cycloheptyl, cyclooctyl, cyclononyl or cyclodecyl, or a bicyclic group such as bicycloheptyl or bicyclooctyl. Different numbers of carbon atoms may be specified, with the definition being amended accordingly.

As used herein, the term "alkoxy" refers to alkyl-O-, wherein alkyl is defined herein above. Representative examples of alkoxy include, methoxy ethoxy, propoxy, 2-propoxy, butoxy, *tert*-butoxy, pentyloxy, hexyloxy, cyclopropyloxy-, cyclohexyloxy-. Typically, alkoxy groups have about 1-7, more suitably about 1-4 carbons.

As used herein, the term "heterocycloalkyl" refers to a saturated or unsaturated non-aromatic ring or ring system, e.g., which is a 4-, 5-, 6-, or 7-membered monocyclic, 7-, 8-, 9-, 10-, 11-, or 12-membered bicyclic or 10-, 11-, 12-, 13-, 14- or 15-membered tricyclic ring system and contains at least one heteroatom selected from O, S and N, where the N and S can also optionally be oxidized to various oxidation states. The heterocyclic group can be attached at a heteroatom or a carbon atom. A C-linked heterocyclic group can be attached at a carbon atom. Examples of heterocycles include tetrahydrofuran (THF), dihydrofuran, 1, 4-dioxane, morpholine, 1,4-dithiane, piperazine, piperidine, 1,3-dioxolane, imidazolidine, imidazoline, pyrroline, pyrrolidine, tetrahydropyran, dihydropyran, oxathiolane, dithiolane, 1,3-dioxane, 1,3-dithiane, oxathiane, thiomorpholine, homomorpholine.

Throughout this specification and in the claims that follow, unless the context requires otherwise, the word "comprise", or variations such as "comprises" or "comprising", should be understood to imply the inclusion of a stated integer or step or group of integers or steps but not the exclusion of any other integer or step or group of integers or steps. The compounds of the invention include compounds of formula (I), and salts thereof as hereinafter defined, polymorphs, isomers and solvates thereof (including optical, geometric and tautomeric isomers) as hereinafter defined and isotopically-labelled compounds of formula (I).

The invention includes also pharmaceutically acceptable salts of a compound of Formula (I). A "pharmaceutically acceptable salt" is intended to mean a salt of a free acid or base of a compound represented by Formula (I),that is non-toxic, biologically tolerable, or otherwise biologically suitable for administration to a subject. See, generally, G.S. Paulekuhn, et al., "Trends in Active Pharmaceutical Ingredient Salt Selection based on Analysis of the Orange Book Database", J. Med. Chem., 2007, 50:6665-72, S.M. Berge, et al., "Pharmaceutical Salts", J Pharm Sci., 1977, 66:1 -19, and Handbook of Pharmaceutical Salts, Properties, Selection, and Use, Stahl and Wermuth, Eds., Wiley-VCH and VHCA, Zurich, 2002.

Examples of pharmaceutically acceptable salts are those that are pharmacologically effective and suitable for contact with the tissues of subjects without undue toxicity, irritation, or allergic response. A compound of Formula (I), may possess a sufficiently acidic group, a sufficiently basic group, or both types of functional groups, and accordingly react with a number of inorganic or organic bases, and inorganic and organic acids, to form a pharmaceutically acceptable salt.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, , hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate , trifluoroacetate and trifluoromethylsulfonate salts.

Inorganic acids from which salts can be derived include, for example, hydrochloric acid, hydrobromic acid, sulfuric acid, nitric acid, phosphoric acid.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, trifluoromethylsulfonic acid, or sulfosalicyclic.

Pharmaceutically acceptable base addition salts can be formed with inorganic and organic bases. Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins. Certain organic amines include isopropylamine, benzathine, cholinate, diethanolamine, diethylamine, lysine, meglumine, piperazine and tromethamine.

Examples of pharmaceutically acceptable salts particularly include sulfates, pyrosulfates, bisulfates, sulfites, bisulfites, phosphates, monohydrogen- phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1 ,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1 -sulfonates, naphthalene-2-sulfonates, and mandelates.

Additionally, any formula given herein is intended to refer also to hydrates, solvates, and polymorphs of such compounds, and mixtures thereof, even if such forms are not listed explicitly. A compound of Formula (I), or pharmaceutically acceptable salt of a compound of Formula (I) may be obtained as a solvate. Solvates include those formed from the interaction or complexation of compounds of the invention with one or more solvents, either in solution or as a solid or crystalline form. In some embodiments, the solvent is water and then the solvates are hydrates. In addition, certain crystalline forms of a compound of Formula (I), or a pharmaceutically acceptable salt of a compound of Formula (I), may be obtained as co-crystals. In certain embodiments of the invention, a compound of Formula (I), or a pharmaceutically acceptable salt of a compound of Formula (I), may be obtained in a crystalline form. In other embodiments, a compound of Formula (I), may be obtained in one of several polymorphic forms, as a mixture of crystalline forms, as a polymorphic form, or as an amorphous form. In other embodiments, a compound of Formula (I), may convert in solution between one or more crystalline forms and/or polymorphic forms.

Compounds of the invention that contain groups capable of acting as donors and/or acceptors for hydrogen bonds may be capable of forming co-crystals with suitable co-crystal formers. These co-crystals may be prepared from compounds of formula (I) by known co-crystal forming procedures. Such procedures include grinding, heating, co-subliming, co-melting, or contacting in solution compounds of formula (I) with the co-crystal former under crystallization conditions and isolating co-crystals thereby formed. Suitable co-crystal formers include those described in WO 2004/078163. Hence the invention further provides co-crystals comprising a compound of formula (I).

Any formula given herein is intended to represent compounds having structures depicted by the structural formula as well as certain forms. In particular, compounds of any formula given herein may have asymmetric centres and therefore exist in different enantiomeric forms. All optical isomers and stereoisomers of the compounds of the general formula, and mixtures thereof, are considered within the scope of the formula. Thus, any formula given herein is intended to represent a racemate, one or more enantiomeric forms, one or more diastereomeric forms, one or more atropisomeric forms, and mixtures thereof. Furthermore, certain structures may exist as geometric isomers (i.e., *cis* and *trans* isomers), as tautomers, or as atropisomers.

Included within the scope of the claimed compounds of the present invention are all stereoisomers, geometric isomers and tautomeric forms of the compounds of Formula (I), including compounds exhibiting more than one type of isomerism, and mixtures of one or more thereof. Also included are acid addition or base addition salts wherein the counter ion is optically active, for example, D-lactate or L-lysine, or racemic, for example, DL-tartrate or DL-arginine.

Where a compound of Formula (I) contains for example, a keto or guanidine group or an aromatic moiety, tautomeric isomerism ('tautomerism') can occur. It follows that a single compound may exhibit more than one type of isomerism. Examples of types of potential tautomerisms shown by the compounds of the invention include; amide ⇔ hydroxyl-imine and keto ⇔ enol tautomerisms:

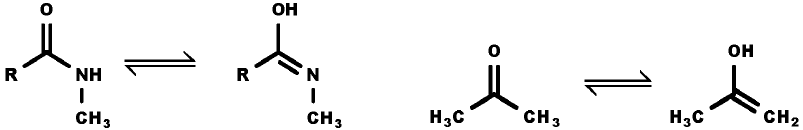

*Cis*/*trans* isomers may be separated by conventional techniques well known to those skilled in the art, for example, by chromatography and fractional crystallisation.

Conventional techniques for the preparation/isolation of individual enantiomers include chiral synthesis from a suitable optically pure precursor or resolution of the racemate (or the racemate of a salt or other derivative) using, for example, chiral high pressure liquid chromatography (HPLC).

Chiral compounds of the invention (and chiral precursors thereof) may be obtained in enantiomerically-enriched form using chromatography, typically HPLC, on a resin with an asymmetric stationary phase and with a mobile phase consisting of a hydrocarbon, typically heptane or hexane, containing from 0 to 50% ethanol, typically from 2 to 20%. Concentration of the eluate affords the enriched mixture.

Mixtures of stereoisomers may be separated by conventional techniques known to those skilled in the art (see, for example, "Stereochemistry of Organic Compounds" by E L Eliel (Wiley, New York, 1994)).

As used herein, the term "isomers" refers to different compounds that have the same molecular formula but differ in arrangement and configuration of the atoms. Also as used herein, the term "an optical isomer" or "a stereoisomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non- superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers or axes and may thus give rise to enantiomers, diastereomers, and other stereoisomeric forms that may be defined, in terms of absolute stereochemistry, as (R)- or (S)-. The present invention is meant to include all such possible isomers, including racemic mixtures, optically pure forms and intermediate mixtures. Optically active (R)- and (S)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration. All tautomeric forms are also intended to be included. Tautomers are one of two or more structural isomers that exist in equilibrium and are readily converted from one isomeric form to another.

Examples of tautomers include for instance those compounds defined in the claims.

Any asymmetric atom (e.g., carbon or the like) of the compound(s) of the present invention can be present in racemic or enantiomerically enriched, for example the (R)-, (S)- or (R,S)- configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration. Substituents at atoms with unsaturated bonds may, if possible, be present in *cis-* (*Z*)- or *trans-* (*E*)- form.

Accordingly, as used herein a compound of the present invention can be in the form of one of the possible stereoisomers or geometric isomers, rotamers, atropisomers, tautomers or mixtures thereof, for example, as substantially pure geometric (*cis* or *trans*) isomers, diastereomers, optical isomers (antipodes), racemates or mixtures thereof.

Any resulting mixtures of isomers can be separated on the basis of the physicochemical differences of the constituents, into the pure or substantially pure geometric or optical isomers, diastereomers, racemates, for example, by chromatography and/or fractional crystallization.

Any resulting racemates of final products or intermediates can be resolved into the optical antipodes by known methods, e.g., by separation of the diastereomeric salts thereof, obtained with an optically active acid or base, and liberating the optically active acidic or basic compound. In particular, a basic moiety may thus be employed to resolve the compounds of the present invention into their optical antipodes, e.g., by fractional crystallization of a salt formed with an optically active acid, *e*.*g*., tartaric acid, dibenzoyl tartaric acid, diacetyl tartaric acid, di-*O*,*O'*-*p*-totuoyl tartaric acid, mandelic acid, malic acid or camphor-10-sulfonic acid. Racemic products can also be resolved by chiral chromatography, *e*.*g*., high pressure liquid chromatography (HPLC) using a chiral adsorbent.

Since the compounds of the invention are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1 %, more suitably at least 5% and preferably from 10 to 59% of a compound of the invention.

When both a basic group and an acid group are present in the same molecule, the compounds of the present invention may also form internal salts, e.g., zwitterionic molecules.

Any formula given herein is also intended to represent unlabelled forms as well as isotopically labelled forms of the compounds. Isotopically labelled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, and fluorine, such as ²H, ³H, ¹¹C, ¹³C, ¹⁴C, ¹³N, ¹⁵N, ¹⁵O, ¹⁷O, ¹⁸O, ¹⁸F, respectively. Such isotopically labelled compounds are useful in metabolic studies (preferably with ¹⁴C), reaction kinetic studies (with, for example ²H or ³H), detection or imaging techniques (such as positron emission tomography (PET) or single-photon emission computed tomography (SPECT)) including drug or substrate tissue distribution assays, or in radioactive treatment of subjects. Substitution with positron emitting isotopes, such as ¹¹C, ¹⁸F, ¹⁵O and ¹³N, can be useful in PET studies for examining substrate receptor occupancy. In particular, an ¹⁸F or ¹¹C labelled compound may be particularly preferred for PET studies. Further, substitution with heavier isotopes such as deuterium (i.e., ²H) may afford certain therapeutic advantages resulting from greater metabolic stability, for example increased *in vivo* half-life or reduced dosage requirements. Certain isotopically-labelled compounds of formula (I) for example, those incorporating a radioactive isotope, are useful in drug and/or substrate tissue distribution studies. The radioactive isotopes tritium, *i*.*e*. ³H, and carbon-14, *i*.*e*. ¹⁴C, are particularly useful for this purpose in view of their ease of incorporation and ready means of detection.

Isotopically labelled compounds of this invention can generally be prepared by carrying out the procedures disclosed in the schemes or in the examples and preparations described below by substituting a readily available isotopically labelled reagent for a non-isotopically labelled reagent.

Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of a compound of the formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

Pharmaceutically acceptable solvates in accordance with the invention include those wherein the solvent of crystallization may be isotopically substituted, *e*.*g*. D₂O, d₆-acetone, d₆-DMSO.

Exemplary compounds of the invention will now be described by reference to the illustrative synthetic schemes for their general preparation below and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be necessary or desirable to employ, in the place of the ultimately desired substituent, a suitable group that may be carried through the reaction scheme and replaced as appropriate with the desired substituent. Unless otherwise specified, the variables are as defined above in reference to Formula (I). Reactions may be performed between the melting point and the reflux temperature of the solvent, and preferably between 0 °C and the reflux temperature of the solvent. Reactions may be heated employing conventional heating or microwave heating. Reactions may also be conducted in sealed pressure vessels above the normal reflux temperature of the solvent.

All of the derivatives of Formula (I) can be prepared by the procedures described in the general methods presented below or by routine modifications thereof. The present application discloses any one or more of these processes for preparing the derivatives of Formula (I), in addition to any novel intermediates used therein.

The routes below, including those mentioned in the Examples and Preparations, illustrate methods of synthesising the compound of Formula (I). The skilled person will appreciate that the compound of the invention, and intermediates thereto, could be made by methods other than those specifically described herein, for example by adaptation of the methods described herein, for example by methods known in the art. Suitable guides to synthesis, functional group interconversions, use of protecting groups, etc., are for example: "Comprehensive Organic Transformations" by RC Larock, VCH Publishers Inc. (1989); "Advanced Organic Chemistry" by J. March, Wiley Interscience (1985); "Designing Organic Synthesis" by S Warren, Wiley Interscience (1978); "Organic Synthesis - The Disconnection Approach" by S Warren, Wiley Interscience (1982); "Guidebook to Organic Synthesis" by RK Mackie and DM Smith, Longman (1982); "Protective Groups in Organic Synthesis" by TW Greene and PGM Wuts, Fifth Ed, John Wiley and Sons, Inc. (2014); and "Protecting Groups" by PJ, Kocienski, Georg Thieme Verlag (1994); and any updated versions of these standard works.

In addition, the skilled person will appreciate that it may be necessary or desirable at any stage in the synthesis of compounds of the invention to protect one or more sensitive groups, so as to prevent undesirable side reactions. In particular, it may be necessary or desirable to protect phenol or carboxylic acid groups. The protecting groups used in the preparation of the compounds of the invention may be used in a conventional manner. See, for example, those described in 'Greene's Protective Groups in Organic Synthesis' by Theodora W Greene and Peter G M Wuts, fifth edition, (John Wiley and Sons, 2014), in particular Chapter 3 ("Protection for Phenols") and Chapter 5 ("Protection for the Carboxyl group*")*, which also describes methods for the removal of such groups.

In the general synthetic methods below, unless otherwise specified, the substituents are as defined above with reference to the compound of Formula (I), above.

Where ratios of solvents are given, the ratios are by volume.

The skilled person will appreciate that the experimental conditions set forth in the schemes that follow are illustrative of suitable conditions for effecting the transformations shown, and that it may be necessary or desirable to vary the precise conditions employed for the preparation of the compound of Formula (**I**). It will be further appreciated that it may be necessary or desirable to carry out the transformations in a different order from that described in the schemes, or to modify one or more of the transformations, to provide the desired compound of the invention.

Compounds prepared according to the schemes described above may be obtained as single enantiomers, diastereomers, or regioisomers, by enantio- , diastero-, or regiospecific synthesis, or by resolution. Compounds prepared according to the schemes above may alternately be obtained as racemic (1:1) or non-racemic (not 1:1) mixtures or as mixtures of diastereomers or regioisomers. Where racemic and non-racemic mixtures of enantiomers are obtained, single enantiomers may be isolated using conventional separation methods known to one skilled in the art, such as chiral chromatography, recrystallization, diastereomeric salt formation, derivatization into diastereomeric adducts, biotransformation, or enzymatic transformation. Where regioisomeric or diastereomeric mixtures are obtained, single isomers may be separated using conventional methods such as chromatography or crystallization.

The compounds of the invention may be prepared by any method known in the art for the preparation of compounds of analogous structure. In particular, the compound of the invention can be prepared by the procedures described by reference to the Schemes that follow, or by the specific methods described in the Examples, or by similar processes to either.

The skilled person will appreciate that the experimental conditions set forth in the schemes that follow are illustrative of suitable conditions for effecting the transformations shown, and that it may be necessary or desirable to vary the precise conditions employed for the preparation of the compound of Formula (I). It will be further appreciated that it may be necessary or desirable to carry out the transformations in a different order from that described in the schemes, or to modify one or more of the transformations, to provide the desired compound of the invention A compound of Formula (**I**) may be prepared from compounds of Formulae (II), (III), (IV) and (V) as illustrated by **Scheme 1**.

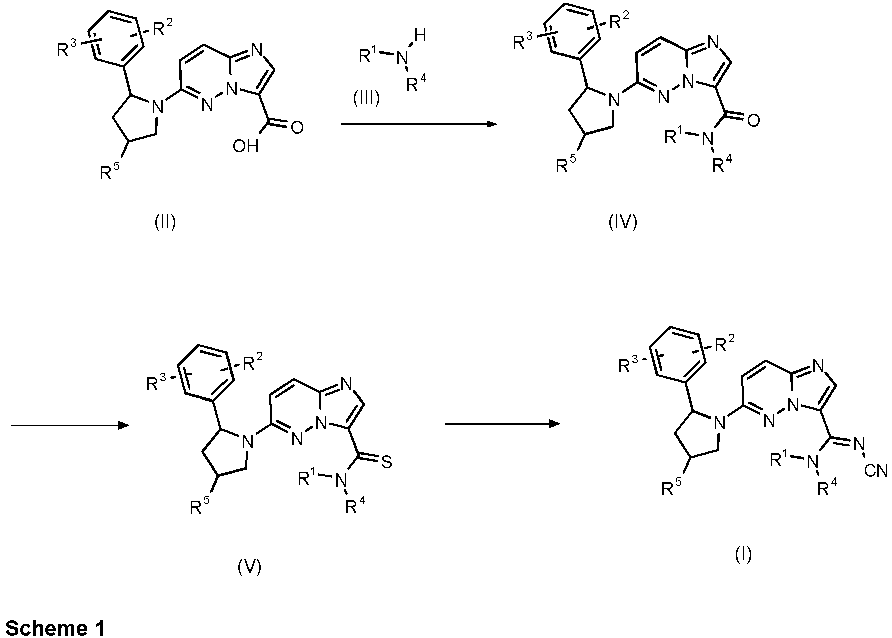

The amine of Formula (III) is commercially available or may be prepared by analogy to methods known in the literature.

The compound of Formula (IV) may be prepared by an amide bond formation of the acid of Formula (II) and the amine of Formula (III) in the presence of a suitable coupling agent and organic base in a suitable polar aprotic solvent. Preferred conditions, comprise the reaction of the acid of Formula (II) with the amine of Formula (III) in the presence of HATU, in the presence of a suitable organic base, typically DIPEA in a suitable solvent, such as DMF at room temperature.

The compound of Formula (V) may be prepared by the thionation of the amide of Formula (IV) using a suitable thionating agent, such as phosphorous pentasulfide or Lawesson's reagent in a suitable solvent. Preferred conditions comprise treatment of the amide of Formula (IV) with Lawesson's reagent in a suitable solvent such as toluene at elevated temperature, such as 100°C.

The compound of Formula (I) may be prepared by treatment of the thioamide of Formula (V) with cyanamide in the presence of a suitable metal catalyst, optionally in the presence of an organic base such as Et₃N or DIPEA in a suitable solvent. Preferred conditions comprise, treatment with cyanamide, in the presence of mercury (II) chloride with Et₃N in a solvent such as DMF at room temperature. Alternatively, this transformation may be achieved by treatment of the thioamide of Formula (V) with cyanamide in the presence of a suitable silver catalyst, such as AgOAc in a solvent such as MeOH at room temperature.

Compounds of Formula (I)(A), wherein R¹ is XR⁷, may be prepared from compounds of Formulae (IV)(A), (VI), (VII) and (VIII) as illustrated in **Scheme 2.**

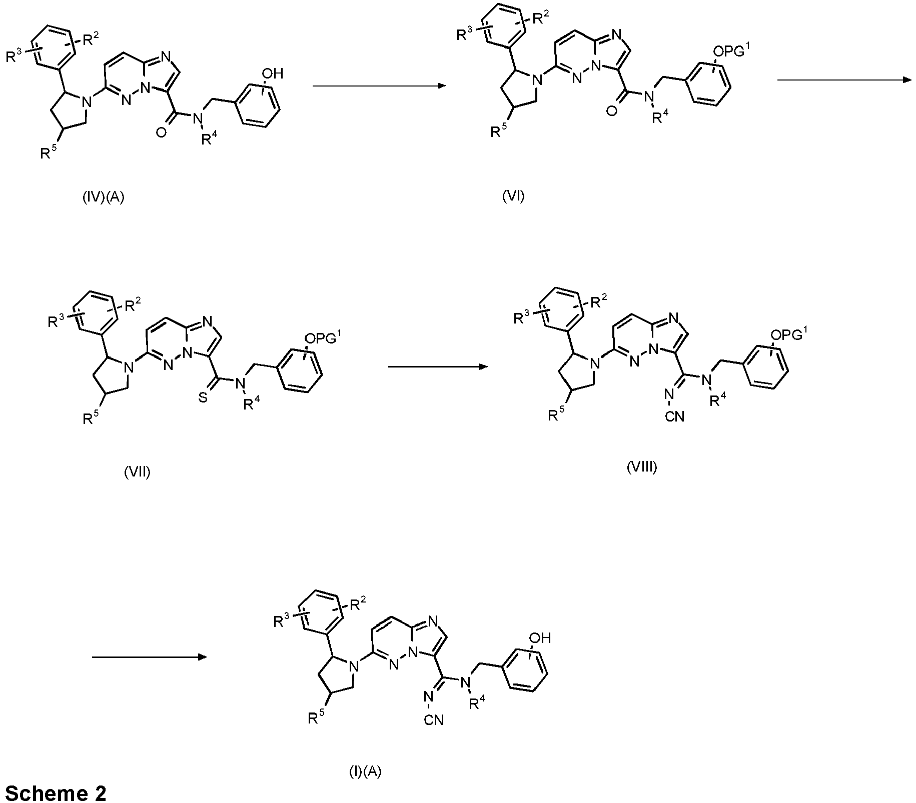

PG¹ is a suitable phenol protecting group, typically a silyl ether group and preferably TBDMS.

The compound of Formula (VI) may be prepared by the protection of the compound of Formula (IV)(A), using a suitable silyl protecting group in a suitable solvent. Preferred conditions comprise treatment of the compound of Formula (IV)(A) with TBDMSCI, in the presence of excess imidazole in DMF at room temperature.

The compound of Formula (VII) may be prepared by the thionation of the compound of Formula (VI) as described in Scheme 1, for the preparation of the compound of Formula (V).

The compound of Formula (VIII) may be prepared by the treatment of the compound of Formula (VII) with cyanamide as described in Scheme 1, for the preparation of the compound of Formula (I).

The compound of Formula (I)(A) may be prepared by the deprotection of the compound of Formula (VIII) under acidic conditions, or in the presence of a tetra-alkylammonium fluoride salt in a suitable solvent. Preferred conditions, comprise treatment of the compound of Formula (VIII) with TEAF in MeCN at elevated temperatures, such as 50°C.

A compound of Formula (IV) may be prepared from the compounds of Formulae (III), (IX), (X) and (XI) as illustrated by **Scheme 3**.

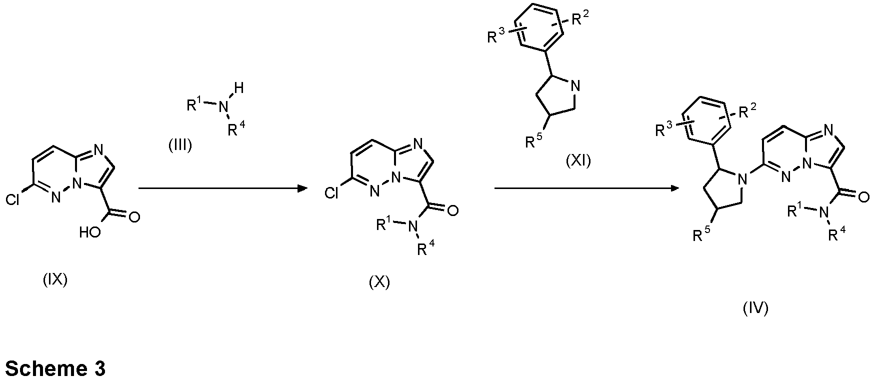

A compound of Formula (IV)(A)(a compound of Formula (IV) wherein R¹ is XR⁷) may also be prepared as illustrated by **Scheme 3**.

The compound of Formula (IX) is commercially available.

Compounds of Formula (XI) are commercially available or may be prepared in chiral form by analogy with the methods described by Brinner et. al. (Org. Biomol. Chem., 2005,3, 2109-2113) or Fan et.al. (WO2012 034091). Alternatively, compounds of Formula (VIII) may be prepared by analogy with the methods described by Huihui et. al. (J. Am. Chem. Soc., 2016, 138, 5016-5019). Alternatively they may be prepared as described in Scheme 5 below.

The amide of formula (X) may be prepared by an amide bond formation of the acid of Formula (IX) and the amine of Formula (III) in the presence of a suitable coupling agent and organic base, as previously described in Scheme 1. Preferred conditions comprise reaction of the acid of Formula (IX) with the amine of Formula (III) in the presence of HATU, in the presence of a suitable organic base, typically DIPEA, in DMF at room temperature.

The compound of Formula (IV) may be prepared by treatment of the compound of Formula (X) with the amine of Formula (XI), in the presence of an inorganic base in a polar aprotic solvent at elevated temperature. Preferred conditions, comprise treatment of the compound of Formula (X) with the amine of Formula (XI) in the presence of KF in a solvent such as DMSO at elevated temperature, typically 130°C.

A compound of Formula (**II**) may be prepared from compounds of Formulae (XI), (XII), and (XIII) as illustrated by **Scheme 4.**

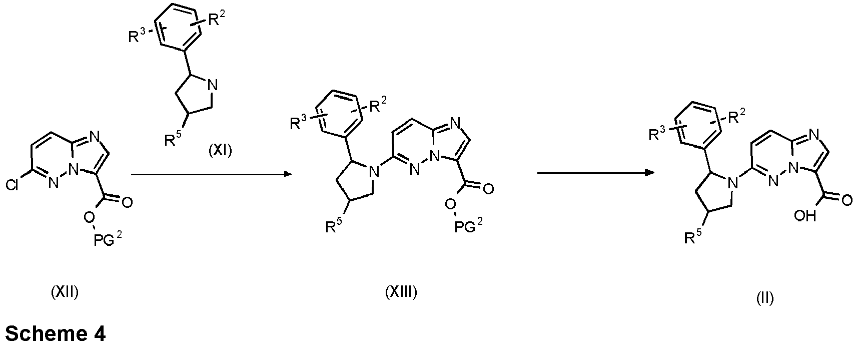

PG² is a carboxyl protecting group, typically a C₁-C₃ alkyl, preferably, ethyl

The compound of Formula (XII) is commercially available or may be prepared by analogy with the methods described by Fan et.al. (WO2012 034091).

The compound of Formula (XIII) may be prepared by treatment of the chloride of Formula (XII) with the amine of Formula (XI), in the presence of an inorganic base in a polar aprotic solvent at elevated temperature. Preferred conditions comprise treatment of the chloride of Formula (XII) with the amine of Formula (XI) in the presence of KF in a solvent such as DMSO at elevated temperature, typically 130°C.

The compound of Formula (II) may be prepared by the hydrolysis of the ester of Formula (XIII) under suitable acidic or basic conditions in a suitable aqueous solvent. Preferred conditions comprise the treatment of the ester of Formula (XIII) with excess NaOH or KOH in aqueous EtOH at room temperature.

A compound of Formula (XI) may be prepared from compounds of Formulae (XIV) (XV) and (XVI) as illustrated by **Scheme 5**

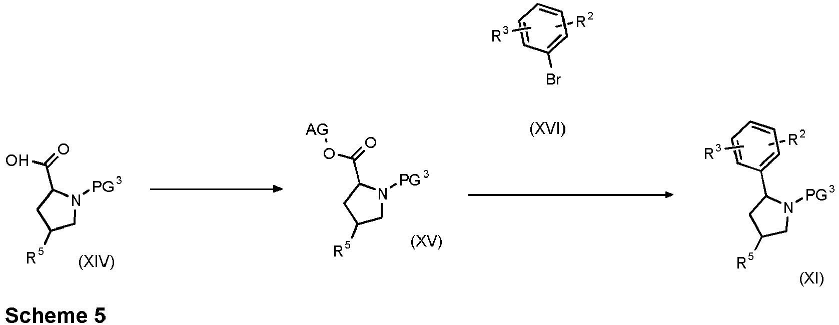

PG³ is a N-protecting group, typically a carbamate or benzyl group, preferably Boc. AG is an activating group, typically a phthalimide, benzotriazole or 7-azabenzotriazole and preferably a phthlimide group.

The compound of Formula (XIV) is commercially available or may be prepared by analogy with known literature methods.

The compound of Formula (XVI) is commercially available or may be prepared by analogy with known literature methods.

The compound of Formula (XV) may be prepared by a coupling reaction of the acid of Formula (XIV) with AG-OH in the presence of a suitable coupling agent. Preferred conditions comprise reaction of the acid of Formula (XIV) with AG-OH in the presence of DCC in EtOAc at room temperature.

The compound of Formula (XI) may be prepared in a two-step Fe or Ni catalysed cross coupling reaction from the bromide of Formula (XVI), via the formation of an intermediate Grignard reagent then treatment with the compound of Formula (XV), following the methods of Toriyama et al (J. Am. Chem. Soc. 2016, 138, 11132-35). Preferred conditions comprise treatment of the bromide of Formula (XVI) with Mg turnings in the presence of DIBAL-H and LiCI in THF at between 0°C and room temperature to prepare the intermediate Grignard reagent. Treatment with the compound of Formula (XV) with a suitable Fe catalyst such as Fe(acac)₃ or Ni(Br)₂ in a suitable polar aprotic solvent(s) such as THF and DMPU at low temperature, typically 0°C.

The above general schemes may be used to prepare compounds of the present invention.The desired specific compounds can be prepared by selecting the appropriate starting materials, reactants and reaction conditions.

The starting materials and reagents in the above scheme are all either available commercially or can be prepared following literature precedents.

Within the scope of this text, only a readily removable group that is not a constituent of the particular desired end product of the compounds of the present invention is designated a "protecting group", unless the context indicates otherwise. The protection of functional groups by such protecting groups, the protecting groups themselves, and their cleavage reactions are described for example in standard reference works, such as 'Greene's Protective Groups in Organic Synthesis' by Theodora W Greene and Peter G M Wuts, fifth edition, (John Wiley and Sons, 2014), in particular Chapter 3 ("Protection for Phenols") and Chapter 5 ("Protection for the Carboxyl group*")*, which also describes methods for the removal of such groups , in J. F. W. McOmie, "Protective Groups in Organic Chemistry", Plenum Press, London and New York 1973, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/l, Georg Thieme Verlag, Stuttgart 1974, in H.-D. Jakubke and H. Jeschkeit, "Aminosäuren, Peptide, Proteine" (Amino acids, Peptides, Proteins), Verlag Chemie, Weinheim, Deerfield Beach, and Basel 1982, and in Jochen Lehmann, "Chemie der Kohlenhydrate: Monosaccharide und Derivate" (Chemistry of Carbohydrates: Monosaccharides and Derivatives), Georg Thieme Verlag, Stuttgart 1974. A characteristic of protecting groups is that they can be removed readily (i.e. without the occurrence of undesired secondary reactions) for example by solvolysis, reduction, photolysis or alternatively under physiological conditions (e.g. by enzymatic cleavage).

Salts of compounds of the present invention having at least one salt-forming group may be prepared in a manner known to those skilled in the art. For example, salts of compounds of the present invention having acid groups may be formed, for example, by treating the compounds with metal compounds, such as alkali metal salts of suitable organic carboxylic acids, e.g. the sodium salt of 2-ethylhexanoic acid, with organic alkali metal or alkaline earth metal compounds, such as the corresponding hydroxides, carbonates or hydrogen carbonates, such as sodium or potassium hydroxide, carbonate or hydrogen carbonate, with corresponding calcium compounds or with ammonia or a suitable organic amine, stoichiometric amounts or only a small excess of the salt-forming agent preferably being used. Acid addition salts of compounds of the present invention are obtained in customary manner, e.g. by treating the compounds with an acid or a suitable anion exchange reagent. Internal salts of compounds of the present invention containing acid and basic salt-forming groups, e.g. a free carboxy group and a free amino group, may be formed, e.g. by the neutralisation of salts, such as acid addition salts, to the isoelectric point, e.g. with weak bases, or by treatment with ion exchangers. Salts can be converted into the free compounds in accordance with methods known to those skilled in the art. Metal and ammonium salts can be converted, for example, by treatment with suitable acids, and acid addition salts, for example, by treatment with a suitable basic agent.

Mixtures of isomers obtainable according to the invention can be separated in a manner known to those skilled in the art into the individual isomers; diastereoisomers can be separated, for example, by partitioning between polyphasic solvent mixtures, recrystallisation and/or chromatographic separation, for example over silica gel or by e.g. medium pressure liquid chromatography over a reversed phase column, and racemates can be separated, for example, by the formation of salts with optically pure salt-forming reagents and separation of the mixture of diastereoisomers so obtainable, for example by means of fractional crystallisation, or by chromatography over optically active column materials.

Intermediates and final products can be worked up and/or purified according to standard methods, e.g. using chromatographic methods, distribution methods, (re-) crystallization, and the like.

The following applies in general to all processes mentioned herein before and hereinafter.

All the above-mentioned process steps can be carried out under reaction conditions that are known to those skilled in the art, including those mentioned specifically, in the absence or, customarily, in the presence of solvents or diluents, including, for example, solvents or diluents that are inert towards the reagents used and dissolve them, in the absence or presence of catalysts, condensation or neutralizing agents, for example ion exchangers, such as cation exchangers, e.g. in the H+ form, depending on the nature of the reaction and/or of the reactants at reduced, normal or elevated temperature, for example in a temperature range of from about -100 °C to about 190 °C, including, for example, from approximately -80 °C to approximately 150 °C, for example at from -80 to -60 °C, at room temperature, at from -20 to 40 °C or at reflux temperature, under atmospheric pressure or in a closed vessel, where appropriate under pressure, and/or in an inert atmosphere, for example under an argon or nitrogen atmosphere.

At all stages of the reactions, mixtures of isomers that are formed can be separated into the individual isomers, for example diastereoisomers or enantiomers, or into any desired mixtures of isomers, for example racemates or mixtures of diastereoisomers, for example analogously to the methods described under "Additional process steps".

The solvents from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofuran or dioxane, liquid aromatic hydrocarbons, such as benzene or toluene, alcohols, such as methanol, ethanol or 1- or 2-propanol, nitriles, such as acetonitrile, halogenated hydrocarbons, such as methylene chloride or chloroform, acid amides, such as dimethylformamide or dimethyl acetamide, bases, such as heterocyclic nitrogen bases, for example pyridine or *N*-methylpyrrolidin-2-one, carboxylic acid anhydrides, such as lower alkanoic acid anhydrides, for example acetic anhydride, cyclic, linear or branched hydrocarbons, such as cyclohexane, hexane or isopentane, methycyclohexane, or mixtures of those solvents, for example aqueous solutions, unless otherwise indicated in the description of the processes. Such solvent mixtures may also be used in working up, for example by chromatography or partitioning.

The compounds, including their salts, may also be obtained in the form of hydrates, or their crystals may, for example, include the solvent used for crystallization. Different crystalline forms may be present.

The invention relates also to those forms of the process in which a compound obtainable as an intermediate at any stage of the process is used as starting material and the remaining process steps are carried out, or in which a starting material is formed under the reaction conditions or is used in the form of a derivative, for example in a protected form or in the form of a salt, or a compound obtainable by the process according to the invention is produced under the process conditions and processed further in situ.

All starting materials, building blocks, reagents, acids, bases, dehydrating agents, solvents and catalysts utilized to synthesize the compounds of the present invention are either commercially available or can be produced by organic synthesis methods known to one of ordinary skill in the art (Houben-Weyl 4th Ed. 1952, Methods of Organic Synthesis, Thieme, Volume 21).

A process for the preparation of compounds of formula I or a pharmaceutically acceptable salt or solvate thereof is disclosed.

Accordingly, there is provided a process of preparing a compound of the present invention or a pharmaceutically acceptable salt or solvate thereof comprising the step of:
treatment of a thioamide of Formula (V) 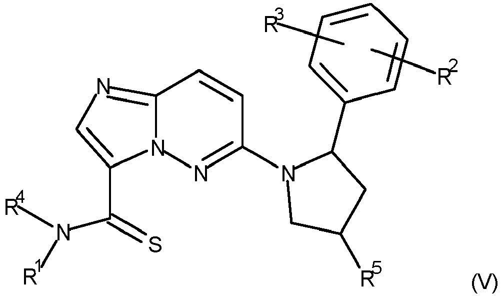 with cyanamide in the presence of a suitable metal catalyst, optionally in the presence of an organic base such as Et₃N or DIPEA in a suitable solvent, wherein R¹, R², R³, R⁴ and R⁵ are as defined anywhere hereinabove in respect of a compound of Formula I.

Moreover, there is provided a process of preparing a compound of the present invention or a pharmaceutically acceptable salt or solvate thereof wherein R¹ is XR⁷ comprising the step of:
deprotection of the compound of Formula (XIII) 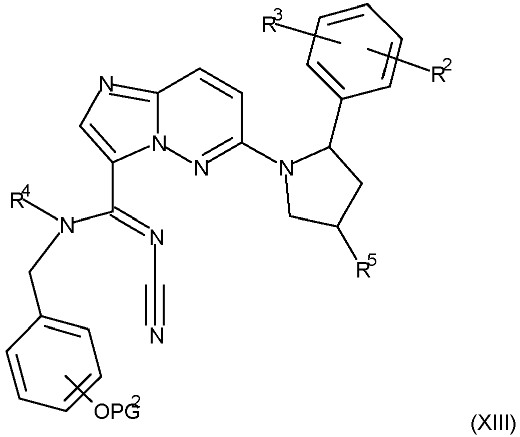 under acidic conditions, or in the presence of an tetraalkylammonium fluoride salt in a suitable solvent, wherein R², R³, R⁴ and R⁵ are as defined anywhere hereinabove in respect of a compound of Formula I and PG² is a protecting group.

Any variant of the present processes are included, in which an intermediate product obtainable at any stage thereof is used as starting material and the remaining steps are carried out, or in which the starting materials are formed *in situ* under the reaction conditions, or in which the reaction components are used in the form of their salts or optically pure antipodes.

Compounds of the invention and intermediates can also be converted into each other according to methods generally known to those skilled in the art.

The compounds of the invention exhibit valuable pharmacological properties, e.g. Trk modulating properties, e.g. as indicated in in vitro and in vivo tests as provided in the next sections and are therefore indicated for therapy.

Having regard to their ability to inhibit Trk activity, the compounds of the invention, hereinafter alternately referred to as "agents of the invention", are useful in the treatment or prevention of a condition or disorder which is mediated by Trk.

In particular, the compounds of the present invention are useful for the treatment of disorders or conditions mediated by the high affinity neurotrophin receptors TrkA, TrkB and TrkC, and the actions of their cognate neurotrophin ligands - NGF, BDNF/NT-4/5, NT-3 - on these receptor tyrosine kinases. Particularly the compounds are useful for treating or preventing conditions of skin (dermal) inflammation and itch (pruritus) that are mediated by the high affinity neurotrophin receptors TrkA, TrkB and TrkC, and associated with inflammation and nerve hypersensitivity, in particular atopic dermatitis.

Infiltration and activation of immune cells in the skin (including T-cell, mast cells, eosinophils) play a key role in inflammatory skin pathologies (Ilkovitch D. J Leukoc Biol. 2011, 89(1):41-9; Kim et al, Int J Mol Sci. 2016,17(8)). TrkA, B, and C and their cognate endogenous neurotrophin ligands have been demonstrated to play a role in the immunological and neurogenic mechanisms associated with skin pathologies (Botchkarev et al, J Invest Dermatol. 2006, 126(8):1719-27.; Truzzi et al, Dermatoendocrinol. 2011, 3(1):32-6; Minnone et al, Int J Mol Sci. 2017, 11;18(5)), and mediate inflammatory functions of skin resident immune cells, particularly those involved in atopic dermatitis pathology (Raap et al, Clin Immunol. 2005, (5):419-24), including T-cells (Sekimoto et al, Immunol Lett. 2003, 88(3):221-6; Matsumura et al, J Dermatol Sci. 2015,78(3):215-23), mast cells (Quarcoo et al, J Occup Med Toxicol. 2009, Apr 22;4:8.), and eosinophils (Raap et al, J Allergy Clin Immunol. 2005, 115:1268-75; Raap et al, Clin Exp Allergy.2008, 38(9):1493-8).

NGF, BDNF, NT-3 and NT-4/5 levels are higher in the lesional skin cells and plasma of atopic dermatitis patients compared to normal subjects and levels correlate with disease severity (Yamaguchi et al, J Dermatol Sci. 2009, 53(1):48-54; Toyoda et al, Br J Dermatol 2002, 147:71-79; Raap et al, J Allergy Clin Immunol. 2005, 115:1268-75; Raap et al, Allergy. 2006, 61 (12): 1416-8). Trk levels are also upregulated in atopic dermatitis lesional skin cells (Dou et al, Arch Dermatol Res. 2006, (1):31-7; Raap et al, Clin Exp Allergy. 2008, 38(9): 1493-8). In addition, the high affinity neurotrophin receptors and their endogenous ligands, in particular Trk A/NGF have been shown to sensitize primary afferent nerves and mediate dermal hyperinnervation, thereby contributing to peripheral itch sensitization and pruritus in particular in atopic dermatitis (Tominaga et al, J Dermatol. 2014, 41(3):205-12; Roggenkamp D et al, J Invest Dermatol 2012, 132: 1892-1900; Grewe et al, J Invest Dermatol 2000, 114:1108-1112). In preclinical mouse models of atopic dermatitis, inhibition of Trk signalling with small molecule compounds that have Trk inhibitory activity, reduced dermatitis and scratching behaviour, with concomitant decreases in nerve fibres in the epidermis (Takano et al, Br J Dermatol. 2007, 156(2):241-6; Narayanan et al, PLoS One. 2013, 26;8(12)).

The compounds of the present invention may be used for the treatment or prevention of skin pathologies or conditions including diseases of dermatitis such as atopic dermatitis (eczema), contact dermatitis, allergic dermatitis; diseases of pruritus such as urticaria (Rössing et al, Clin Exp Allergy. 2011, 41(10):1392-9), Cutaneous T-cell lymphoma (CTCL) -associated pruritus including Sezary syndrome (Suga et al, Acta Derm Venereol. 2013, 93(2):144-9; Saulite et al, Biomed Res Int. 2016 doi: 10.1155/2016/9717530); Psoriasis (Raychaudhuri et al, Prog Brain Res. 2004, 146:433-7); diseases of skin pain and neuropathy (Hirose et al, Pain Pract. 2016, 16(2):175-82; Wang et al, J Neurosci. 2009, 29(17):5508-15).

In particular, conditions or disorders which are mediated by Trk, in particular Trk A, B, and C, include, diseases of pruritus and itch; autoimmune diseases of the skin; diseases of skin pain and neuropathy; and diseases of dermatitis.

Diseases of pruritus and itch include, skin diseases, eczematous dermatitis, atopic; eczema ;dermatitis, contact; dermatitis, allergic contact; dermatitis, irritant; dermatitis, photoallergic ; dermatitis, phototoxic ; psoriasis ; pruritus; pruritus ani; pruritus, hereditary localized; Sjogrens syndrome associated pruritis; idiopathic pruritus; sclerosis multiplex pruritus; prurigo nodularis; brachioradial pruritus; acute itch; chronic itch; diabetes pruritus; iron deficiency anaemia pruritus; polycythemia vera pruritus; graft-versus-host-disease ; uraemic pruritus; cholestatic pruritus; pruritic urticarial papules and plaques of pregnancy; pemphigoid gestationis; senile pruritus; HIV associated pruritus; shingles; herpes zoster oticus; larva migrans; tinea corporis; tungiasis; exanthema; Fox-Fordyce disease; skin diseases, parasitic; skin diseases, bacterial; cutaneous T-cell; lymphoma-associated pruritus; Sezary syndrome; mycosis fungoides; colorectal cancer; melanoma; head and neck cancer; drug eruption pruritus (iatrogenic); drug reactions; urticarial; vibratory urticarial; physical urticarial; familial cold urticarial; allergic urticarial; dermatographia; dermatitis herpetiformis; Grover disease.

Autoimmune diseases of the skin include, autoimmune disease of skin and connective tissue; autoimmune disease with skin involvement; autoimmune bullous skin disease; pemphigoid, bullous.

Diseases of skin pain and neuropathy include diabetic neuropathies ; neuralgia; painful neuropathy ; nerve compression syndromes ; neuritis; sensory peripheral neuropathy ; alcoholic neuropathy ; radiculopathy ; complex regional pain syndromes ; polyneuropathy due to drug; plantar nerve lesion; polyradiculopathy; sciatic neuropathy;trigeminal neuralgia.

Diseases of dermatitis include, skin diseases, eczematous dermatitis, atopic ; eczema ; dermatitis, contact; dermatitis, allergic contact; dermatitis, irritant; dermatitis, photoallergic ; dermatitis, phototoxic; chronic irritative hand dermatitis; dermatitis, occupational ; fiberglass dermatitis ; dermatitis, toxicodendron ; eczema, dyshidrotic; eczematous dermatitis of eyelid; allergic contact dermatitis of eyelid; hand and foot dermatitis; digital dermatitis; dermatitis, exfoliative; radiodermatitis; dermatitis herpetiformis ; juvenile dermatitis herpetiformis ; autoimmune progesterone dermatitis ; dermatitis, seborrheic; pityriasis lichenoides; blepharitis; nummular dermatitis ; Seborrhea-Like Dermatitis with Psoriasiform Elements; infective dermatitis associated with HTLV-1; psoriasis; generalized pustular psoriasis; skin diseases, papulosquamous; parapsoriasis; keratosis; hyperkeratosis, epidermolytic; skin sarcoidosis; skin atrophy; erythematosquamous dermatosis; poikiloderma with neutropenia; erythema multiforme; angiolymphoid hyperplasia with eosinophilia; keratosis palmoplantaris striata 3; acne vulgaris; lamellar ichthyosis; lichen disease; lichen planus; actinic lichen planus; lichen planus, oral; lichen planus follicularis ; lichen sclerosus et atrophicus ; lichen nitidus; lichen sclerosus; lichen simplex chronicus; scleroderma, limited; keratosis linearis with ichthyosis congenita and sclerosing keratoderma; erythrokeratoderma, reticular; keratosis palmoplantaris papulose; skin diseases, genetic; autosomal recessive congenital ichthyosis; autosomal recessive congenital ichthyosis 1; autosomal recessive congenital ichthyosis 2; autosomal recessive congenital ichthyosis 3 autosomal recessive congenital ichthyosis 4A; autosomal recessive congenital ichthyosis 5; autosomal recessive congenital ichthyosis 6; autosomal recessive congenital ichthyosis 7; autosomal recessive congenital ichthyosis 8 autosomal recessive congenital ichthyosis 9; autosomal recessive congenital ichthyosis 10; autosomal recessive congenital ichthyosis 11.

More particularly, the condition or disorder which is mediated by Trk, in particular Trk A, B, and C, may be atopic dermatitis.

Treatment in accordance with the invention may be symptomatic or prophylactic,

Thus in a further aspect the invention includes an agent of the invention for use as a pharmaceutical.

Therefore according to a further aspect, the invention provides an agent of the invention for treating or preventing a condition or disorder which is mediated by Trk, in particular Trk A, B, and C.

In another aspect the invention provides an agent of the invention for preventing or treating a condition or disorder which is mediated by Trk, in particular Trk A, B, and C, more particularly atopic dermatitis.

As referred to herein a "disorder" or a "disease" refers to an underlying pathological disturbance in a symptomatic or asymptomatic organism relative to a normal organism, which may result, for example, from infection or an acquired or congenital genetic imperfection.

A "condition" refers to a state of the mind or body of an organism which has not occurred through disease, e.g. the presence of a moiety in the body such as a toxin, drug or pollutant.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both. In yet another embodiment, "treat", "treating" or "treatment" refers to preventing or delaying the onset or development or progression of the disease or disorder.

"Prevention" of a condition or disorder refers to delaying or preventing the onset of a condition or disorder or reducing its severity, as assessed by the appearance or extent of one or more symptoms of said condition or disorder.

As used herein, the term "subject" refers to an animal. Typically the animal is a mammal. A subject also refers to for example, primates (e.g., humans), cows, sheep, goats, horses, dogs, cats, rabbits, rats, mice, fish, birds and the like. In certain embodiments, the subject is a primate. In yet other embodiments, the subject is a human.

As used herein, a subject is "in need of" a treatment if such subject would benefit biologically, medically or in quality of life from such treatment.

The term "a therapeutically effective amount" of an agent of the invention refers to an amount of the agent of the invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the agent of the invention that, when administered to a subject, is effective to at least partially alleviating, inhibiting, preventing and/or ameliorating a condition or disorder which is mediated by TrK, in particular Trk A, B, and C. In another non-limiting embodiment, the term "a therapeutically effective amount" refers to the amount of the agent of the invention that, when administered to a cell, or a tissue, or a non-cellular biological material, or a medium, is effective to at least partially inhibiting Trk activity, in particular Trk A, B, and C.

In one embodiment of the present invention, the condition or disorder which is mediated by Trk, in particular Trk A, B, and C, is selected from diseases of pruritus and itch; autoimmune diseases of the skin; diseases of skin pain and neuropathy; and diseases of dermatitis.

In a particular embodiment, the condition or disorder which is mediated by Trk, in particular Trk A, B, and C, is atopic dermatitis.

As described above, the agents of the invention, which inhibit Trk, in particular Trk A, B, and C, have various clinical applications and thus a further aspect of the invention provides pharmaceutical compositions containing agents of the invention. The use of these agents as a medicament forms a further aspect of the invention.

The active agents of the invention are used, alone or in combination with one or more additional active ingredients, to formulate pharmaceutical compositions of the invention. A pharmaceutical composition of the invention comprises: (a) an effective amount of at least one active agent in accordance with the invention; and (b) a pharmaceutically acceptable excipient.

Thus, in a further aspect the present invention provides a pharmaceutical composition comprising an agent of the invention and a pharmaceutically acceptable excipient. Pharmaceutical compositions as described herein for use as a medicament, in particular for use in treating or preventing disorders or conditions medited by Trk, in particular Trk A, B, and C, such as the conditions described herein, and use of said agents for the preparation of a medicament for treating or preventing such disorders or conditions, form further aspects of the invention.

"Pharmaceutically acceptable" as referred to herein refers to ingredients that are compatible with other ingredients of the compositions as well as physiologically acceptable to the recipient.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of an agent and that is compatible therewith. Examples of excipients include calcium carbonate, calcium phosphate, various sugars and types of starch, cellulose derivatives, gelatin, vegetable oils, and polyethylene glycols.

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

Pharmaceutical compositions according to the invention may be formulated in conventional manner using readily available ingredients. Thus, the active ingredient may be incorporated, optionally together with other active substances, with one or more conventional carriers, diluents and/or excipients, to produce conventional galenic preparations such as tablets, pills, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, sterile packaged powders, and the like.

The pharmaceutical composition can be formulated for particular routes of administration such as oral administration, parenteral administration, and rectal administration, etc. In addition, the pharmaceutical compositions of the present invention can be made up in a solid form (including without limitation capsules, tablets, pills, granules, powders or suppositories), or in a liquid form (including without limitation solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers, etc.

Typically, the pharmaceutical compositions are tablets or gelatin capsules comprising the active ingredient together with
a) diluents, e.g., lactose, polylactone, dextrose, sucrose, mannitol, sorbitol, cellulose and/or glycine;
b) lubricants, e.g., silica, talcum, stearic acid, its magnesium or calcium salt and/or polyethylene glycol; for tablets also
c) binders, e.g., magnesium aluminum silicate, starch paste, gelatin, tragacanth, methylcellulose, sodium carboxymethylcellulose and/or polyvinylpyrrolidone; if desired
d) disintegrants, e.g., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of an agent of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Suitable compositions for topical application to the skin or mucosa (e.g., to the skin and eyes), that is dermally or transdermally, include aqueous solutions, suspensions, ointments, creams, gels, hydrogels, microemulsions, dusting powders, dressings, foams, films, skin patches, wafers, implants, fibres, bandages or sprayable formulations, e.g., for delivery by aerosol or the like. Such topical delivery systems will in particular be appropriate for dermal application, e.g., for the treatment of atopic dermatitis. They are thus particularly suited for use in topical, including cosmetic, formulations well-known in the art. Such may contain solubilizers, stabilizers, tonicity enhancing agents, buffers and preservatives. Typical carriers include alcohol, water, mineral oil, liquid petrolatum, white petrolatum, glycerin, polyethylene glycol and propylene glycol. Penetration enhancers may be incorporated [see, for example, Finnin and Morgan, J Pharm Sci, 88 (10), 955-958 (October 1999).]

Suitable compositions for transdermal application include an effective amount of an agent of the invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

Dosages of agents of the invention employed in practising the present invention will of course vary depending, for example, on the particular condition to be treated, the effect desired and the mode of administration. In general, suitable daily dosages for administration by inhalation are of the order of 0.0001 to 30 mg/kg, typically 0.01 to 10 mg per patient, while for oral administration suitable daily doses are of the order of 0.01 to 100 mg/kg.

The present invention further provides anhydrous pharmaceutical compositions and dosage forms comprising the agents of the invention as active ingredients, since water may facilitate the degradation of certain compounds.

Anhydrous pharmaceutical compositions and dosage forms of the invention can be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

The agent of the invention may be administered either simultaneously with, or before or after, one or more other therapeutic agent. The agent of the invention may be administered separately, by the same or different route of administration, or together in the same pharmaceutical composition as the other agents.

In one embodiment, the invention provides a product comprising an agent of the invention and at least one other therapeutic agent as a combined preparation for simultaneous, separate or sequential use in therapy. In one embodiment, the therapy is the treatment of a condition or disorder which is mediated by Trk, in particular Trk A, B, and C. Products provided as a combined preparation include a composition comprising the agent of the invention and the other therapeutic agent(s) together in the same pharmaceutical composition, or the agent of the invention and the other therapeutic agent(s) in separate form, e.g. in the form of a kit.

In one embodiment, the invention provides a pharmaceutical composition comprising an agent of the invention and another therapeutic agent(s). Optionally, the pharmaceutical composition may comprise a pharmaceutically acceptable excipient, as described above.

In one embodiment, the invention provides a kit comprising two or more separate pharmaceutical compositions, at least one of which contains an agent of the invention. In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and topical, for administering the separate compositions at different dosage intervals, or for titrating the separate compositions against one another. To assist compliance, the kit of the invention typically comprises directions for administration.

In the combination therapies of the invention, the agent of the invention and the other therapeutic agent may be manufactured and/or formulated by the same or different manufacturers. Moreover, the agent of the invention and the other therapeutic may be brought together into a combination therapy: (i) prior to release of the combination product to physicians (e.g. in the case of a kit comprising the agent of the invention and the other therapeutic agent); (ii) by the physician themselves (or under the guidance of the physician) shortly before administration; (iii) in the patient themselves, e.g. during sequential administration of the agent of the invention and the other therapeutic agent.

Accordingly, the invention provides the use of an agent of the invention for treating a condition or disorder which is mediated by Trk, in particular Trk A, B, and C, wherein the medicament is prepared for administration with another therapeutic agent. The invention also provides the use of another therapeutic agent for treating a condition or disorder which is mediated by Trk, in particular Trk A, B, and C, wherein the medicament is administered with an agent of the invention.

The combination may serve to increase efficacy (e.g., by including in the combination a compound potentiating the potency or effectiveness of an active agent according to the invention), decrease one or more side effects, or decrease the required dose of the active agent according to the invention.

The invention also provides an agent of the invention for use in a method of treating a condition or disorder which is mediated by Trk, in particular Trk A, B, and C, wherein the agent of the invention is prepared for administration with another therapeutic agent. The invention also provides another therapeutic agent for use in a method of treating a condition or disorder which is mediated by Trk, in particular Trk A, B, and C, wherein the other therapeutic agent is prepared for administration with an agent of the invention. The invention also provides an agent of the invention for use in a method of treating a condition or disorder which is mediated by Trk, in particular Trk A, B, and C, wherein agent of the invention is administered with another therapeutic agent. The invention also provides another therapeutic agent for use in a method of treating a condition or disorder which is mediated by Trk, in particular Trk A, B, and C, wherein the other therapeutic agent is administered with an agent of the invention.

The invention also provides the use of an agent of the invention for treating a condition or disorder which is mediated by Trk, in particular Trk A, B, and C, wherein the subject has previously (e.g. within 24 hours) been treated with another therapeutic agent. The invention also provides the use of another therapeutic agent for treating a condition or disorder which is mediated by Trk, in particular Trk A, B, and C, wherein the subject has previously (e.g. within 24 hours) been treated with an agent of the invention.

In one embodiment, a compound of the invention is administered alongside one or more other therapeutically active agents. For example, the compounds of the invention may therefore be used in combination with one or more further agents for the treatment of atopic dermatitis, such as: one or more topical and/or oral corticosteroids; one or more antihistamines; one or more antibiotics; one or more topical calcineurin inhibitors such as tacrolimus and/or pimecrolimus; one or more systemic immunosuppressants such as cyclosporin, methotrexate, interferon gamma-1b, mycophenolate mofetil and/or azathioprine; one or more PDE4 inhibitors such as crisaborole; one or more monoclonal antibodies such as dupilumab.

A skilled person will appreciate that an agent of the invention may be administered to a subject, particularly a human subject, wherein the subject is being treated with phototherapy for a condition or disorder which is mediated by Trk, in particular Trk A, B, and C, such as atopic dermatitis. A compound of the invention may also be administered to a subject, particularly a human subject, wherein the subject has previously (e.g. within 24 hours) been treated with phototherapy for a condition or disorder in which is mediated by Trk, in particular Trk A, B, and C, such as atopic dermatitis. A subject, particularly a human subject may also be treated with phototherapy for a condition or disorder which is mediated by Trk, in particular Trk A, B, and C, such as atopic dermatitis wherein a compound of the invention has previously (e.g. within 24 hours) been administered to a subject.

Accordingly, the invention includes as a further aspect a combination of an agent of the invention with one or more further agents for the treatment of atopic dermatitis, such as: one or more topical and/or oral corticosteroids; one or more antihistamines; one or more antibiotics; one or more topical calcineurin inhibitors such as tacrolimus and/or pimecrolimus; one or more systemic immunosuppressants such as cyclosporin, methotrexate, interferon gamma-1b, mycophenolate mofetil and/or azathioprine; one or more PDE4 inhibitors such as crisaborole; one or more monoclonal antibodies such as dupilumab; and phototherapy.

### In vitro assays

A suitable assay for determining the Trk inhibition activity of a compound is detailed herein below.

To determine the IC₅₀ of small molecule compounds for the Human TRK receptors, HTRF^{®} KinEASE^{™} kinase kits from Cisbio were used. Assays were carried out in low volume, black 384-well plates.

Recombinant Human TRK enzymes (Invitrogen) were incubated in the presence or absence of the compound (11-point dose response with FAC as 10µM) for 30 minutes at 23°C. Kinase reaction was started by addition of ATP to a mixture containing the enzyme (NTRK1-4nM, NTRK2-1nM, NTRK3-10nM) and substrate (1µM). Kinase reaction was allowed to carry on for 10 to 45 minutes at 23°C after which it was stopped by addition of the detection mix (supplied by vendor) containing EDTA, TK-Ab- labelled with Eu³⁺-cryptate (1:200 dilutions) and Streptavidin-XL665 (250nM). Assay plates were incubated in this detection mix for 60 minutes at 23°C. The resulting TR-FRET signal, calculated as the fluorescence ratio at 665/620 nm, was read on an Envision and was proportional to the level of phosphorylation of the peptide in the presence or absence of the compound. The uniformity of the plates were assured with Z' value [1-{3*(SDHPE+SDZPE)/ (ZPE-HPE)}]. The percent (%) effect i.e. Inhibition of compound was calculated in comparison to the signal in the positive (HPE) and negative control (ZPE) wells within each assay plate. The endpoint value % Inhibition for the Standard compound was evaluated in each experiment as a quality control measure. IC₅₀ was determined by plotting compound inhibition at respective dose in Graphpad prism5 using four parameter logistic curve fit.

Using the assay described above, the compounds of the present invention all exhibit of Trk inhibition activity, expressed as an IC₅₀ value, of less than 1 µM. Preferred examples have IC₅₀ values of less than 200nM and particularly preferred examples have IC₅₀ values of less than 50nM. IC₅₀ values for the compounds of Examples 1, 2, 3, 4, 5, 6 and 7 are given below in Table 1.

**Table 1: Trk inhibition activity, expressed as IC₅₀ values**

| Example | **TrkA Enz (nM)** | **TrkB Enz (nM)** | **TrkC Enz (nM)** |
|---|---|---|---|
| 1 | 0.95 | 0.88 | 1.60 |
| 2 | 1.04 | 0.83 | 1.78 |
| 3 | 1.17 | 0.37 | 1.53 |
| 4 | 0.97 | 0.23 | 1.00 |
| 5 | 1.10 | 0.41 | 1.61 |
| 6 | 1.52 | 0.70 | 1.91 |
| 7 | 1.44 | 1.55 | 3.16 |

### EXAMPLES

Referring to the examples that follow, compounds of the preferred embodiments are synthesized using the methods described herein, or other methods, which are known in the art.

It should be understood that the organic compounds according to the preferred embodiments may exhibit the phenomenon of tautomerism. As the chemical structures within this specification can only represent one of the possible tautomeric forms, it should be understood that the preferred embodiments encompasses any tautomeric form of the drawn structure.

It is understood that the invention is not limited to the embodiments set forth herein for illustration, but embraces all such forms thereof as come within the scope of the above disclosure.

### General Conditions:

The following examples are intended to illustrate the invention and are not to be construed as being limitations thereon. Temperatures are given in degrees centigrade. If not mentioned otherwise, all evaporations are performed under reduced pressure. The structure of final products, intermediates and starting materials is confirmed by standard analytical methods, e.g., microanalysis and spectroscopic characteristics, e.g., MS, IR, NMR. Abbreviations used are those conventional in the art. If not defined, the terms have their generally accepted meanings.

Abbreviations and acronyms used herein include the following:

| **Abbreviation/acronym** | **Term** |
|---|---|
| AcOH | Acetic acid |
| AgOAc | Silver acetate |
| aq | aqueous |
| Bn | benzyl |
| br | broad |
| °C | degrees Celsius |
| CDCl₃ | deutero-chloroform |
| Cs₂CO₃ | Cesium carbonate |
| Cy | cyclohexane |
| δ | chemical shift |
| d | doublet |
| dd | double doublet |
| ddd | Doublet of doublets of doublets |
| DCC | N,N'-dicyclohexylcarbodiimide |
| DCM | dichloromethane |
| DIBAL-H | Diisobutylaluminium hydride |
| DIPEA | N-ethyldiisopropylamine or N,N-diisopropylethylamine |
| DMAP | 4-(dimethylamino)pyridine |
| DMF | N,N-dimethylformamide |
| DMPU | N,N'-dimethylpropylene urea |
| DMSO | Dimethylsulfoxide |
| DMSO-d₆ | hexadeuterodimethyl sulfoxide |
| Et | ethyl |
| Et₃N | triethylamine |
| EtOH | ethanol |
| EtOAc | ethyl acetate |
| Fe(acac)₃ | Iron (III) acetylacetone |
| g | gram |
| HCI | hydrochloric acid |
| HATU | (1-[Bis(dimethylamino)methylene]-1H-1,2,3-triazolo[4,5-b]pyridinium 3-oxid hexafluorophosphate) |
| H₂O | water |
| HPLC | high pressure liquid chromatography |
| Hr | hour |
| IPA | Isopropyl alcohol |
| KF | Potassium fluoride |
| KOH | Potassium hydroxide |
| L | litre |
| Lawesson's Reagent | 2,4-Bis(4-methoxyphenyl)-2,4-dithioxo-1,3,2,4-dithiadiphosphetane |
| LCMS | liquid chromatography mass spectrometry |
| LiCI | Lithium chloride |
| m | multiplet |
| M | molar |
| mBar | millibar |
| Me | methyl |
| MeCN | acetonitrile |
| MeOH | methanol |
| MeOD-d₄ | deutero-methanol |
| 2-MeTHF | 2-methyltetrahydrofuran |
| mg | milligram |
| MHz | mega Hertz |
| mins | minutes |
| mL | millilitres |
| mmol | millimole |
| MS m/z | mass spectrum peak |
| MsCl | methanesulfonyl chloride |
| MTBE | Methyl *tert*-butyl ether |
| M/V | Mass volume ratio |
| N₂ | nitrogen |
| NaBH₄ | sodium borohydride |
| NaHCO₃ | sodium bicarbonate |
| NaOH | sodium hydroxide |
| NH₃ | ammonia |
| NH₄Cl | ammonium chloride |
| Na₂SO₄ | sodium sulfate |
| PtO₂ | platinum (IV) oxide |
| q | quartet |
| rt | room temperature |
| RT | retention time |
| s | singlet |
| sat. | saturated |
| soln. | solution |
| t | triplet |
| TBDMS | tert-butyldimethylsilyl |
| TBDMSCI | tert-butyldimethylsilyl chloride |
| TEAF | tetraethylammonium fluoride |
| THF | tetrahydrofuran |
| TMS | trimethylsilyl |
| µL | micro litres |
| v/v | volume volume percent |
| w/w | Weight/weight percent |

Referring to the examples that follow, compounds of the preferred embodiments were synthesized using the methods described herein, or other methods, which are known in the art.

The various starting materials, intermediates, and compounds of the preferred embodiments may be isolated and purified, where appropriate, using conventional techniques such as precipitation, filtration, crystallization, evaporation, distillation, and chromatography. Unless otherwise stated, all starting materials are obtained from commercial suppliers and used without further purification. Salts may be prepared from compounds by known salt-forming procedures.

It should be understood that the organic compounds according to the preferred embodiments may exhibit the phenomenon of tautomerism. As the chemical structures within this specification can only represent one of the possible tautomeric forms, it should be understood that the preferred embodiments encompasses any tautomeric form of the drawn structure.

¹H nuclear magnetic resonance (NMR) spectra were in all cases consistent with the proposed structures. Characteristic chemical shifts (δ) are given in parts-per-million downfield from tetramethylsilane (for ¹H-NMR) using conventional abbreviations for designation of major peaks: e.g. s, singlet; d, doublet; t, triplet; q, quartet; m, multiplet; br, broad. The following abbreviations have been used for common solvents: CDCl₃, deuterochloroform; DMSO-d₆, hexadeuterodimethyl sulfoxide; and MeOD-d₄, deuteron-methanol. Where appropriate, tautomers may be recorded within the NMR data; and some exchangeable protons may not be visible.

Mass spectra, MS (m/z), were recorded using either electrospray ionisation (ESI) or atmospheric pressure chemical ionisation (APCI). Where relevant and unless otherwise stated the m/z data provided are for isotopes ¹⁹F, ³⁵Cl, ⁷⁹Br and ¹²⁷I.

Where preparative TLC or silica gel chromatography have been used, one skilled in the art may choose any combination of solvents to purify the desired compound.

Example compounds of the present invention include:

### Example 1

### (Z)-N'-cyano-6-[(2R)-2-[5-fluoro-2-(methylsulfanyl)phenyl]pyrrolidin-1-yl]-N-[(3R)-oxan-3-yl]imidazo[1,2-b]pyridazine-3-carboximidamide

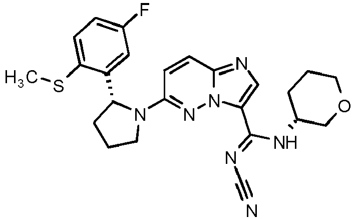

Mercury dichloride (144 mg, 0.530 mmol) was added in one portion, followed by cyanamide (62 mg, 1.48 mmol) to a solution of 6-[(2R)-2-[5-fluoro-2-(methylsulfanyl)phenyl]pyrrolidin-1-yl]-N-[(3R)-oxan-3-yl]imidazo[1,2-b]pyridazine-3-carbothioamide (Preparation 27, 100 mg, 0.212 mmol) in DMF (2 mL) under N₂ and the reaction stirred at rt for 16 hrs. The mixture was concentrated *in vacuo,* the residue resuspended in DCM (10 mL), filtered through a pad of Dicalite^{®}, rinsing through with 20% MeOH in DCM (20 mL). The filtrate was concentrated *in vacuo* and purified by column chromatography on silica gel eluting with heptanes:EtOAc:MeOH, (80:20:0 to 0:100:0 to 0:90:10). The product was triturated with MeOH to afford the title compound as a colourless solid, 30 mg, 30%.

LCMS m/z = 480.2 [M+H]⁺

¹H NMR (DMSO-d₆, 400MHz): δ 1.55-1.57 (m, 1H), 1.80-2.02 (m, 5H), 2.39-2.50 (m, 5H), 3.44-3.73 (m, 3H), 3.76-3.87 (m, 1H), 3.94-4.04 (m, 1H), 4.07-4.20 (m, 1H), 5.26 (d, 1H), 6.40 (br s, 1H), 6.92 (dd, 1H), 7.16 (dd, 1H), 7.41 (dd, 1H), 7.97 (d, 1H), 8.70 (s, 1H), 9.75 (br s, 1H).

### Examples 2 to 3

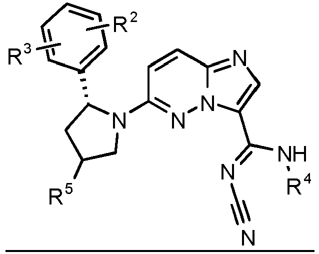

The compounds in the table below were prepared from the appropriate thioamide using the method described in Example 1.

| Example | Structure and name | Starting Materials, Yield and Data |
|---|---|---|
| 2 | 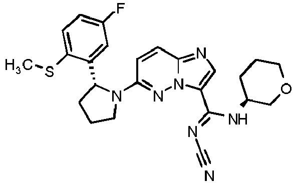 | 6-[(2R)-2-[5-fluoro-2-(methylsulfanyl)phenyl]pyrrolidin-1-yl]-N-[(3S)-oxan-3-yl]imidazo[1,2-b]pyridazine-3-carbothioamide (Preparation 28) |
| | (Z)-N'-cyano-6-[(2R)-2-[5-fluoro-2-(methylsulfanyl)phenyl]pyrrolidin-1-yl]-N-[(3S)-oxan-3-yl]imidazo[1,2-b]pyridazine-3-carboximidamide | 13% as a colourless solid |
| | | LCMS m/z = 480.3 [M+H]⁺ |
| | | ¹HNMR (MeOD-d₄, 400 MHz): δ 1.57-1.70 (m, 1H), 1.90-2.11 (m, 6H), 2.50-2.68 (m, 4H), 3.63-3.73 (m, 1H), 3.74-3.85 (m, 3H), 3.86-3.96 (m, 1H), 4.06-4.17 (m, 1H), 4.22-4.33 (m, 1H), 5.36-5.46 (m, 1H), 6.50-6.60 (m, 1H), 6.80-6.90 (m, 1H), 7.01-7.11 (m, 1H), 7.38-7.48 (m, 1H), 7.78 (d, 1H), 8.85-8.91 (m, 1H). |
| 3 | 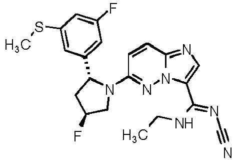 | N-ethyl-6-[(2R,4S)-4-fluoro-2-[5-fluoro-3-(methylsulfanyl)phenyl]pyrrolidin-1-yl]imidazo[1,2-b]pyridazine-3-carbothioamide (Preparation 31) 47% colourless solid yield as a |
| | (Z)-N'-cyano-N-ethyl-6-[(2R,4S)-4-fluoro-2-[5-fluoro-3-(methylsulfanyl)phenyl]pyrrolidin-1-yl]imidazo[1,2-b]pyridazine-3-carboximidamide | LCMS m/z = 441.9 [M+H]⁺ |
| | | ¹HNMR (DMSO-d₆, 400 MHz): δ 1.18 (t, 3H), 2.04-2.22 (m, 1H), 2.47 (s, 3H), 2.80-2.90 (m, 1H), 3.32-3.42 (m, 2H), 4.14-4.26 (m, 2H), 5.29 (t, 1H), 5.32, 5.57 (2x s, 1H), 6.93-7.00 (m, 3H), 7.14 (s, 1H), 8.05 (d, 1H), 8.53 (s, 1H), 9.11 (br s, 1H). |

### Example 4

### (Z)-N'-cyano-N-ethyl-6-[(2R,4S)-4-fluoro-2-[5-fluoro-2-(methylsulfanyl)phenyl]pyrrolidin-1-yl]imidazo[1,2-b]pyridazine-3-carboximidamide

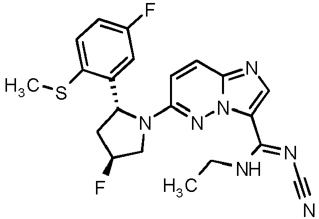

Silver acetate (33 mg, 0.18 mmol) was added to a stirred solution of N-ethyl-6-[(2R,4S)-4-fluoro-2-[5-fluoro-2-(methylsulfanyl)phenyl]pyrrolidin-1-yl]imidazo[1,2-b]pyridazine-3-carbothioamide (Preparation 30, 86 mg, 0.20 mmol), and cyanamide (42 mg, 0.99 mmol) in dry MeOH (2 mL) under N₂. The mixture was stirred for 2 hr at rt, additional silver acetate (33 mg, 0.18 mmol) added and the reaction flask protected from light by covering with aluminium foil. The reaction was stirred for a further 48 hrs, the resulting suspension filtered and the filtrate evaporated to dryness. The residue was purified by column chromatography on silica gel eluting with DCM:MeOH (96:4) and then by reverse phase column chromatography eluting with MeCN:water (5:95 to 95:5)to afford the title compound as a colourless solid, 35 mg, 40%.

¹HNMR (MeOD-d₄, 400 MHz): δ 1.25 (t, 3H), 2.08-2.24 (m, 1H), 2.58 (s, 3H), 3.00-3.10 (m, 1H), 3.48-3.54 (m, 2H), 4.16-4.32 (m, 2H), 5.39-5.59 (m, 2H), 6.92-7.10 (m, 3H), 7.40 (dd, 1H), 7.86 (d, 1H), 8.72 (s, 1H)

LCMS m/z = 442.0 [M+H]⁺

### Example 5

### (Z)-N-butyl-N'-cyano-6-[(2R,4S)-4-fluoro-2-[5-fluoro-2-(methylsulfanyl)phenyl]pyrrolidin-1-yl]imidazo[1,2-b]pyridazine-3-carboximidamide

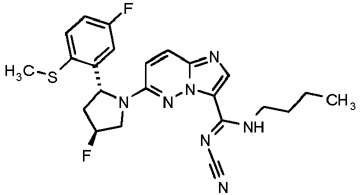

The title compound was obtained as a colourless solid in 43% yield, from of N-butyl-6-[(2R,4S)-4-fluoro-2-[5-fluoro-2-(methylsulfanyl)phenyl]pyrrolidin-1-yl]imidazo[1,2-b]pyridazine-3-carbothioamide (Preparation 32), following the method described in Example 4.

LCMS m/z = 470 [M+H]^{+ 1}H-NMR (MeOD-d₄, 396 MHz) : δ 1.01 (t, 3H), 1.40-1.50 (m, 2H), 1.54-1.71 (m, 2H), 2.10-2.27 (m, 1H), 2.57 (s, 3H), 3.02-3.12 (m, 1H), 3.48 (t, 2H), 4.18-4.30 (m, 2H), 5.39-5.58 (m, 2H), 6.89-7.09 (m, 3H), 7.41 (dd, 1H), 7.83 (d, 1H), 8.70 (s, 1H).

### Example 6

### (Z)-N'-cyano-N-cyclohexyl-6-[(2R,4S)-4-fluoro-2-[5-fluoro-2-(methylsulfanyl)phenyl]pyrrolidin-1-yl]imidazo[1,2-blpyridazine-3-carboximidamide

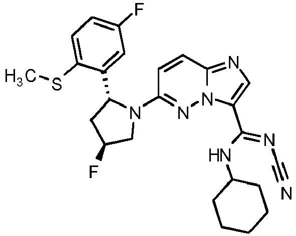

The title compound was obtained as a pale yellow solid in 4% yield, from N-cyclohexyl-6-[(2R,4S)-4-fluoro-2-[5-fluoro-2-(methylsulfanyl)phenyl]pyrrolidin-1-yl]imidazo[1,2-b]pyridazine-3-carbothioamide (Preparation 33), following the method described in Example 4.

LCMS m/z =496 [M+H]⁺

¹HNMR (MeOD-d₄, 400 MHz): δ 1.24-1.57 (m, 5H), 1.67-1.81 (m, 1H), 1.82-1.96 (m, 2H), 2.08-2.34 (m, 3H), 2.58 (s, 3H), 2.88-3.20 (m, 1H), 3.95-4.26 (m, 3H), 5.39-5.66 (m, 2H), 6.64 (d, 1H), 6.96-7.16 (m, 2H), 7.44 (dd, 1H), 7.80 (d, 1H), 8.66 (s, 1H).

### Example 7

### (Z)-N'-cyano-6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboximidamide

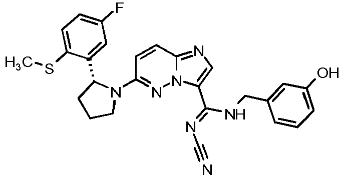

TEAF (73 mg, 0.49 mmol) was added in one portion to a solution of (Z)-N-({3-[(tert-butyldimethylsilyl)methyl]phenyl}methyl)-N'-cyano-6-[(2R)-2-[5-fluoro-2-(methylsulfanyl)phenyl]pyrrolidin-1-yl]imidazo[1,2-b]pyridazine-3-carboximidamide (Preparation 34, 60 mg, 0.097 mmol) in MeCN (0.5 mL) and the reaction stirred at 50 °C for 4 hrs. The cooled mixture was evaporated *in vacuo,* the residue diluted with EtOAc (15 mL), washed with water (3 × 15 mL), the organic phase dried (Na₂SO₄), filtered and evaporated. The residue was purified by column chromatography on silica gel eluting with DCM:MeOH (99:1 to 92:8) and the product triturated with water to afford the title compound, as a colourless solid, 16 mg, 33%.

LCMS m/z = 502.0 [M+H]⁺

¹H NMR (MeOD-d₄, 400MHz): δ 1.89-1.99 (m, 3H), 2.42-2.48 (m, 4H), 3.10-3.30 (m, 2H), 4.53-4.58 (m, 2H), 5.33 (d, 1H), 6.67-6.99 (m, 6H), 7.15-7.25 (m, 2H), 7.80-7.87 (m, 1H), 8.90 (s, 1H).

### Preparation 1

### 4-fluoro-2-iodo-1-(methylsulfanyl)benzene

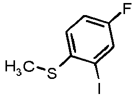

2-Bromo-4-fluoro-1-(methylsulfanyl)benzene (0.5 g, 2.26 mmol) was added dropwise to a suspension of activated Mg turnings (1.92 g, 79 mmol) under N₂(g) in dry THF (80 mL) and the reaction warmed until Grignard formation had initiated. The remaining 2-bromo-4-fluoro-1-(methylsulfanyl)benzene (17 g, 76.89 mmol) was added dropwise, so as to maintain the temperature below 50°C and after complete addition, the reaction was allowed to cool to rt and stirred for 16 hrs. The solution was added via cannula to an ice-cooled solution of iodine (24.11 g, 94.99 mmol) in dry THF (80 mL) maintaining the temperature below 10 °C. The reaction was stirred at 0 °C for 1 hr, at rt for 1 hr then poured into an ice-cold sat. NH₄Cl soln. (300 mL). The mixture was concentrated in *vacuo* to remove organic solvents then extracted with Et₂O (3 × 300 mL). The combined organic layers were washed with a sat. Na₂S₂O₃ soln., dried (Na₂SO₄), and concentrated *in vacuo* to afford the title compound as a brown oil, 21.5 g, 83 %.

¹H NMR (CDCl₃, 396 MHz): δ 2.45 (s, 3H), 7.08-7.11 (m, 2H), 7.55 (dd, 1H).

### Preparation 2

### 1-tert-butyl 2-(1,3-dioxo-2,3-dihydro-1H-isoindol-2-yl) (2S,4S)-4-fluoropyrrolidine-1,2-dicarboxylate

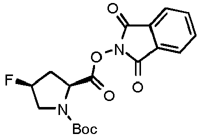

A solution of (2S,4S)-1-(*tert*-butoxycarbonyl)-4-fluoropyrrolidine-2-carboxylic acid (1.07 g, 4.6 mmol) in EtOAc (12.5 mL) was added to a stirred mixture of N-hydroxyphthalimide (0.75 g, 4.6 mmol) and N,N'-dicyclohexylcarbodiimide (0.95 g, 4.6 mmol) in EtOAc (12.5 mL) under N₂(g) and the reaction stirred at rt for 4 hrs. The mixture was filtered through a plug of silica, washed with EtOAc (50 mL) and the filtrate concentrated *in vacuo.* The resulting oil was re-dissolved in EtOAc (20 mL), washed with sat. aq. NaHCO₃ (4 × 30 mL) and the organic layer dried (MgSO₄), filtered and evaporated under reduced pressure to afford the title compound as a white solid, 1.55 g, 89 %.

LCMC m/z = 278.9 [M-Boc]⁺

### Preparation 3

### tert-butyl (2R, 4S)-4-fluoro-2-[5-fluoro-2-(methylsulfanyl)phenyl]pyrrolidine-1-carboxylate

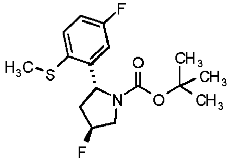

Nickel dibromide ethylene glycol dimethyl ether complex (0.09 g, 0.291 mmol) and 4,4'-di-tert-butyl-2,2'-bipyridine (0.08 g, 0.298 mmol) were flushed with N₂(g) and dry DMA (4 mL) added. The resulting blue-green mixture was stirred under N₂(g) for 15 mins then 4-fluoro-2-iodo-1-(methylsulfanyl)benzene (Preparation 1, 0.51 g, 1.49 mmol), 1-*tert*-butyl 2-(1,3-dioxo-2,3-dihydro-1H-isoindol-2-yl) (2S,4S)-4-fluoropyrrolidine-1,2-dicarboxylate (Preparation 2, 0.62 g, 1.64 mmol) and zinc dust (0.251 g, 3.84 mmol) were added and the reaction was stirred at 28 °C for 17 hr. The reaction mixture was filtered through a plug of silica and washed with Et₂O (75 mL). The filtrate was washed with brine (4 × 75 mL), dried (MgSO₄), filtered and concentrated *in vacuo.* The residue was purified by column chromatography on silica gel eluting with heptanes:EtOAc (100:0 to 90:10) to afford the title compound as a yellow oil, 0.24 g, 36%.

LCMS m/z = 230.1 [M-Boc]⁺

### Preparation 4

### tert-butvl N-[(2R)-2-[(tert-butyldimethylsilyl)oxy]-4-[3-fluoro-5-(methylsulfanyl)phenyl]-4-hydroxybutyl]carbamate

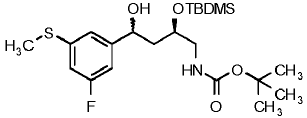

20 mL of a solution of 3-bromo-5-fluoro-1-(methylsulfanyl)benzene (38.0 g, 146 mmol) in dry THF (110 mL) was added dropwise to a stirred suspension of activated Mg turnings (10.7 g, 438 mmol) under N₂(g) in dry THF (110 mL) and the reaction warmed until Grignard formation had initiated. The remaining 3-bromo-5-fluoro-1-(methylsulfanyl)benzene solution was then added so as to maintain the temperature below 50°C. After complete addition, the reaction was allowed to cool to rt and stirred for a further hr. The solution was added via cannula to a -20 °C solution of tert-butyl (R)-4-(tert-butyldimethylsilyloxy)-2-oxopyrrolidine-1-carboxylate (US9701681, Example 6, 38.4 g, 122 mmol) in dry THF (220 mL) so as to maintain the temperature below -10 °C. The mixture was stirred at -50 °C for 1 hr, at 0 °C for 1 hr then re-cooled to -20 °C. MeOH (150 mL) was added dropwise, followed by NaBH₄ (6.91 g, 182 mmol) in 5 portions and the reaction stirred at -15 °C for 30 mins then for 3.5 hrs at rt. The mixture was poured into ice-cold sat. NH₄Cl soln. (150 mL), then concentrated *in vacuo* to remove organic solvents and extracted with EtOAc (3 x 150 mL). The combined organic phases were dried (MgSO₄), evaporated under reduced pressure and the crude product purified by column chromatography on silica gel eluting with heptanes:EtOAc, (95:5 to 60:40) to afford the title compound as a pale yellow oil, 35.8 g, 64 %.

LCMS m/z = 342.4 [M-Boc-H₂O]⁺

### Preparation 5

### tert-butyl (4R)-2-[3-fluoro-5-(methylsulfanyl)phenyl]-4-hydroxypyrrolidine-1-carboxylate

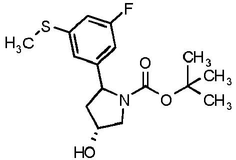

Et₃N (33 mL, 237 mmol) followed by mesyl chloride (9.10 mL, 117 mmol) were added dropwise to an ice cooled solution of tert-butyl N-[(2R)-2-[(tert-butyldimethylsilyl)oxy]-4-[3-fluoro-5-(methylsulfanyl)phenyl]-4-hydroxybutyl]carbamate (Preparation 4, 35.8 g, 77.9 mmol) in anhydrous DCM (210 mL) and the reaction stirred for 2 hrs. The mixture was poured into ice-cold water (140 mL), extracted with DCM (3 x 70 mL) and the combined organic extracts dried (MgSO₄) and concentrated *in vacuo.*

The residue was dissolved in THF (140 mL), TBAF (1M in THF, 110 mL, 110 mmol) added and the reaction stirred at rt for 2 hrs. This was then poured into cold water (200 mL), concentrated *in vacuo* to remove organic solvents and extracted with EtOAc (3 x 150 mL). The combined organic phases were dried (MgSO₄), evaporated under reduced pressure and purified by column chromatography on silica gel eluting with heptanes:EtOAc (95:5 to 0:100) to afford the title compound as a light yellow oil, 23.7 g, 93%.

LCMS m/z = 228 [M-Boc]⁺

### Preparation 6

### tert-butyl (2R, 4S)-4-fluoro-2-[5-fluoro-3-(methylsulfanyl)phenyl]pyrrolidine-1-carboxylate

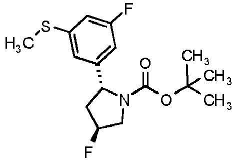

DAST (16.9 mL, 139 mmol) was added dropwise to a -5 °C solution of *tert*-butyl (4R)-2-[3-fluoro-5-(methylsulfanyl)phenyl]-4-hydroxypyrrolidine-1-carboxylate (Preparation 5, 22.74 g, 69.5 mmol) in dry DCM (290 mL) so as to maintain the internal temperature below 0 °C. The reaction mixture was stirred for 2.5 hrs at rt, then poured carefully into an ice-cold sat. aq. NaHCO₃ soln. (250 mL). This mixture was extracted with DCM (3 x 200 mL), the combined organic layers dried (MgSO₄), concentrated *in vacuo* and purified by column chromatography on silica gel eluting with heptanes:TBME (100:0 to 70:30) to provide the title compound as a pale yellow oil, 4.2 g, 18%.

¹H NMR (CDCl₃, 400MHz): δ 1.25 (s, 6H), 1.46 (s, 3H), 2.20-2.36 (m, 1H), 2.45 (s, 3H), 2.48-2.67 (m, 1H), 3.76 (dd, 1H), 3.97 (dd, 1H), 4.81-5.08 (m, 1H), 5.20-5.26 (m, 1H), 6.70-6.76 (m, 1H), 6.76-6.80 (m, 1H), 6.87-6.90 (m, 1H).

### Preparation 7

### 5-fluoro-2-(methylsulfanyl)benzaldehyde

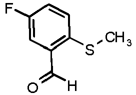

n-BuLi in hexane (2.5 M, 0.4 mL, 1 mmol) was added dropwise to a solution of 2-bromo-4-fluoro-1-(methylsulfanyl)benzene (221.0 mg, 1 mmol) in dry THF (10 mL) at -78 °C under N₂(g), so the temperature was maintained below -70 °C. DMF (80.0 mg, 1.1 mmol) was added and the reaction stirred at -78°C for a further 30 mins. The resulting mixture was quenched by the addition of ice-cold sat. aq. NH₄Cl soln. (10 mL), warmed to rt and extracted with EtOAc (10 mL). The organic extracts were washed with saturated brine (10 mL), dried (MgSO₄), concentrated *in vacuo* and purified by column chromatography on silica gel eluting with heptanes:EtOAc (95:5) to afford the title compound as colourless oil, 88 mg, 52%.

¹H NMR (CDCl₃, 400MHz): δ 2.51 (s, 3H), 7.25-7.30 (m, 1H), 7.35-7.39 (m, 1H), 7.52-7.56 (m, 1H), 10.35 (s, 1H).

### Preparation 8

### (R)-N-[(1Z)-[5-fluoro-2-(methylsulfanyl)phenyl]methylidene]-2-methylpropane-2 sulfinamide

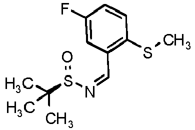

Cs₂CO₃ (300.0 mg, 0.92 mmol) was added to a solution of 5-fluoro-2-(methylthio)benzaldehyde (Preparation 7, 130.0 mg, 0.76 mmol) and (R)-2-methylpropane-2-sulfinamide (93.0 mg, 0.76 mmol) in DCM (15 mL) and the reaction stirred at rt for 18 hrs. Water (15 mL) was carefully added, the phases separated, the organic layer was dried (MgSO₄), and evaporated under reduced pressure. The residue was purified by column chromatography on silica gel eluting with heptanes:EtOAc (95:5 to 85:15) to afford the title compound as a yellow oil, 130 mg, 62%.

LCMS m/z = 274.1 [M+H]⁺

### Preparation 9

### (R)-N-[(1R)-3-(1-[5-fluoro-2-(methylsulfanyl)phenyl]propyl]-2-methylpropane-2-sulfinamide

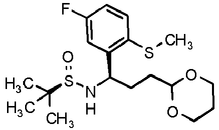

A solution (0.5 mL) of 2-(2-bromoethyl)-1,3-dioxolane (1.81 g, 10 mmol) in dry THF (5 mL) was added to a suspension of activated Mg turnings (729.0 mg, 30.0 mmol) under N₂(g) in dry THF (10 mL) and the reaction warmed until Grignard formation had initiated. The remaining 2-(2-bromoethyl)-1,3-dioxolane solution (4.5 mL) was slowly added maintaining the temperature below 50°C. After complete addition, the reaction mixture was allowed to cool to rt, stirred for a further 1 hr then re-cooled to -50 °C. A solution of (R)-N-[(1Z)-[5-fluoro-2-(methylsulfanyl)phenyl]methylidene]-2-methylpropane-2-sulfinamide (Preparation 8, 270.0 mg, 1 mmol) in dry THF (5 mL) was added dropwise, the reaction stirred at -50°C for 1 hr and then allowed to warm to rt. Sat. aq. NH₄Cl soln. (20 mL) was added to quench the reaction and the mixture partitioned between EtOAc (30 mL) and water (30 mL). The aqueous phase was further extracted with EtOAc (30 mL) and the combined organics washed with brine (60 mL), dried (MgSO₄) and concentrated *in vacuo.* The crude product was purified by column chromatography on silica gel eluting with heptanes:EtOAc, (50:50 to 0:100) to afford the title compound as a colourless oil, 420 mg, 100%.

LCMS m/z = 390.0 [M+H]⁺

### Preparation 10

### (2R)-2-[5-fluoro-2-(methylsulfanyl)phenyl]pyrrolidine

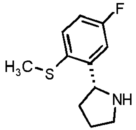

A solution of (R)-N-[(1R)-3-(1,3-dioxan-2-yl)-1-[5-fluoro-2-(methylsulfanyl)phenyl]propyl]-2-methylpropane-2-sulfinamide (Preparation 9, 390.0 mg, 1 mmol) in TFA:water (10 mL, 20:1) was stirred at rt for 30 mins. Et₃SiH (1.16 g, 10 mmol) was added and the reaction stirred vigorously at rt for 16 hrs. The mixture was diluted with toluene (30 mL), concentrated *in vacuo* then azeotroped with toluene (2 × 30 mL). The residual oil was purified by column chromatography on silica gel eluting with (DCM:MeOH:NH₄OH, 98:2:0.2 to 95:5:0.5) to afford the title compound product as an oil 125 mg, 59%.

LCMS m/z = 212.0 [M+H]⁺

### Preparation 11

### (2R,4S)-4-fluoro-2-[5-fluoro-2-(methylsulfanyl)pheny]pyrrolidine

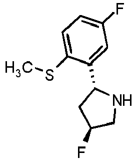

HCI (4M solution in dioxane, 10 mL) was added to a solution of tert-butyl (2R, 4S)-4-fluoro-2-[5-fluoro-2-(methylsulfanyl)phenyl]pyrrolidine-1-carboxylate (Preparation 3, 1.21 g, 3.67 mmol) in MeOH (15 mL) and the reaction stirred at rt for 2 hrs. The mixture was concentrated *in vacuo* to afford a dark brown oil which was dissolved in MeOH (2 mL) and loaded onto an SCX ion exchange cartridge washing through with 7N NH₄OH in MeOH. The filtrate was evaporated under reduced pressure to afford the title compound as a dark orange oil, 0.4 g, 53%.

LCMS m/z = 230.0 [M+H]⁺

### Preparation 12

### (2R,4S)-4-fluoro-2-[5-fluoro-3-(methylsulfanyl)phenyl]pyrrolidine hydrochloride

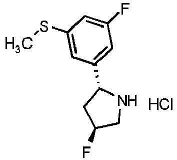

A solution of tert-butyl (2R,4S)-4-fluoro-2-[5-fluoro-3-(methylthio)phenyl]pyrrolidine-1-carboxylate (Preparation 6, 3.88 g, 11.79 mmol) in 4M HCI in dioxane (60 mL) was stirred at rt for 2 hrs. The solution was concentrated *in vacuo* to afford the title compound as a beige solid, 3.69 g, 99%.

LCMS m/z = 230 [M+H]⁺

### Preparation 13

### Ethyl 6-[(2R)-2-[5-fluoro-2-(methylsulfanyl)phenyl]pyrrolidin-1-yl]imidazo[1,2-b]pyridazine-3-carboxylate

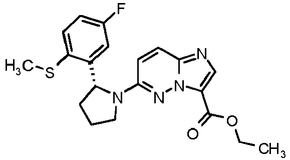

A solution of (2R)-2-[5-fluoro-2-(methylsulfanyl)phenyl]pyrrolidine (Preparation 10, 640 mg, 3.03 mmol) in 4M HCI in dioxane (20 mL) was stirred at rt for 30 mins then concentrated *in vacuo.* Ethyl 6-chloroimidazo[1,2-b]pyridazine-3-carboxylate (0.59 g, 2.52 mmol) in DMSO (20 mL) was added and the reaction heated at 130 °C for 16 hrs. The cooled mixture was partitioned between water (20 mL) and EtOAc (20 mL), and the layers separated. The organic phase was washed with brine (3 × 20mL), dried (MgSO₄) and evaporated under reduced pressure to afford the title compound as a brown oil, 1.13 g, 99%.

LCMS m/z = 401.2 [M+H]⁺

### Preparation 14

### Ethyl 6-[(2R,4S)-4-fluoro-2-[5-fluoro-2-(methylsulfanyl)phenyl]pyrrolidin-1-yl]imidazo[1,2-b]pyridazine-3-carboxylate

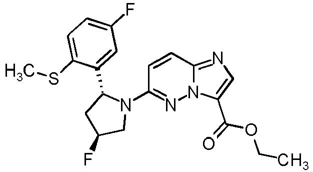

The title compound was obtained as a yellow solid in 85% yield from (2R,4S)-4-fluoro-2-[5-fluoro-2-(methylsulfanyl)phenyl]pyrrolidine (Preparation 11) following the procedure described in Preparation 13.

LCMS m/z = 419.0 [M+H]⁺

### Preparation 15

### Ethyl 6-[(2R,4S)-4-fluoro-2-[5-fluoro-3-(methylsulfanyl)phenyl]pyrrolidin-1-yl]imidazo[1,2-b]pyridazine-3-carboxylate

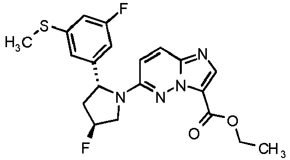

The title compound was obtained as a brown oil in 78% yield from (2R,4S)-4-fluoro-2-[5-fluoro-3-(methylsulfanyl)phenyl]pyrrolidine hydrochloride (Preparation 12) following the procedure described in Preparation 13.

LCMS m/z = 419.0 [M+H]⁺

### Preparation 16

### 6-[(2R)-2-[5-fluoro-2-(methylsulfanyl)phenyl]pyrrolidin-1-yl]imidazo[1,2-b]pyridazine-3-carboxylic acid

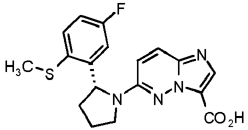

KOH (0.71 g, 12.6 mmol) was added portion wise to a solution of ethyl 6-[(2R)-2-[5-fluoro-2-(methylsulfanyl)phenyl]pyrrolidin-1-yl]imidazo[1,2-b]pyridazine-3-carboxylate (Preparation 13, 1.0 g, 2.52 mmol) in EtOH:water (12 mL, 6:1) and the reaction stirred at rt for 1.5 hrs. The mixture was concentrated *in vacuo,* the residue partitioned between water (20 mL) and DCM (20 mL) and the layers separated. The aqueous phase was adjusted to pH 4 with 2 M HCI solution, then extracted with DCM (3 x 20 mL). These combined organic phases were dried (MgSO₄)and concentrated *in vacuo* to give the title compound as a beige solid, 999 mg, 99%.

LCMS m/z = 373.2 [M+H]⁺

### Preparation 17

### 6-[(2R,4S)-4-fluoro-2-[5-fluoro-2-(methylsulfanyl)phenyl]pyrrolidin-1-yl]imidazo[1,2-b]pyridazine-3-carboxylic acid

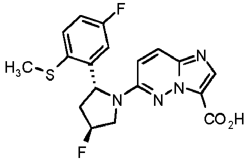

The title compound was obtained as a yellow solid in 50% yield from ethyl 6-[(2R,4S)-4-fluoro-2-[5-fluoro-2-(methylsulfanyl)phenyl]pyrrolidin-1-yl]imidazo[1,2-b]pyridazine-3-carboxylate (Preparation 14) following the procedure described in Preparation 16.

LCMS m/z = 391 [M+H]⁺

### Preparation 18

### 6-[(2R,4S)-4-fluoro-2-[5-fluoro-3-(methylsulfanyl)phenyl]pyrrolidin-1-yl]imidazo[1,2-b]pyridazine-3-carboxylic acid

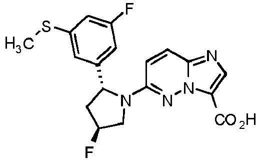

The title compound was obtained as a brown oil in 78% yield from ethyl 6-[(2R,4S)-4-fluoro-2-[5-fluoro-3-(methylsulfanyl)phenyl]pyrrolidin-1-yl]imidazo[1,2-b]pyridazine-3-carboxylate (Preparation 15) following the procedure described in Preparation 16.

LCMS m/z = 391 [M+H]⁺

### Preparation 19

### 6-[(2R)-2-[5-fluoro-2-(methylsulfanyl)phenyl]pyrrolidin-1-yl]-N-[(3R)-oxan-3-yl]imidazo[1,2-b]pyridazine-3-carboxamide

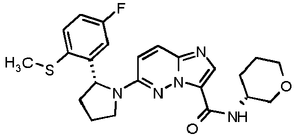

To a solution of 6-[(2R)-2-[5-fluoro-2-(methylsulfanyl)phenyl]pyrrolidin-1-yl]imidazo[1,2-b]pyridazine-3-carboxylic acid (Preparation 16, 150 mg, 0.407 mmol) in DMF (5 mL) was added (R)-tetrahydro-2H-pyran-3-amine hydrochloride (61 mg, 0.443 mmol) and HATU (168 mg, 0.443 mmol). The mixture was stirred at rt for 5 mins, DIPEA (0.140 mL, 0.805 mmol) added and the reaction stirred at rt for a further 16 hrs. The reaction was diluted with EtOAc (15 mL), washed with water (15 mL) and brine (15 mL), then dried (Na₂SO₄) and concentrated *in vacuo.* The crude product was purified by column chromatography on silica gel eluting with DCM:MeOH (99:1 to 92:8) and the product azeotroped with water to afford the title compound as a colourless solid, 133 mg, 72%.

LCMS m/z = 456.2 [M+H]⁺

### Preparations 20 to 24

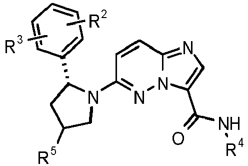

The following compounds were prepared from the appropriate carboxylic acid and amine, R⁴NH₂, following the procedure described in Preparation 19.

| Prepa ration No | Structure and Name | Starting Materials | Yield, Data |
|---|---|---|---|
| 20 | 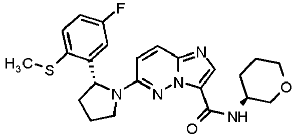 | 6-[(2R)-2-[5-fluoro-2-(methylsulfanyl)phenyl]pyrrolidin -1-yl]imidazo[1,2-b]pyridazine-3-carboxylic acid (Preparation 16) and (S)-tetrahydro-2H-pyran-3-amine hydrochloride | Brown solid, 77% |
| | 6-[(2R)-2-[5-fluoro-2-(methylsulfanyl)phenyl]pyrrolidin-1-yl]-N-[(3S)-oxan-3-yl]imidazo[1,2-b]pyridazine-3-carboxamide | | LCMS m/z = 456 [M+H]⁺ |
| 21 | 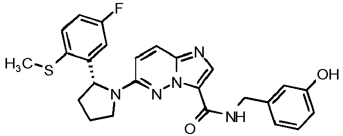 | 6-[(2R)-2-[5-fluoro-2-(methylsulfanyl)phenyl]pyrrolidin -1-yl]imidazo[1,2-b]pyridazine-3-carboxylic acid (Preparation 16) and 3-hydroxybenzylamine hydrochloride | Colourles s solid, 55% |
| | 6-[(2R)-2-[5-fluoro-2-(methylsulfanyl)phenyl]pyrrolidin-1-yl]-N-[(3-hydroxyphenyl)methyl]imidazo[1,2 -b]pyridazine-3-carboxamide | | LCMS m/z = 478.1 [M+H]⁺ |
| 22 | 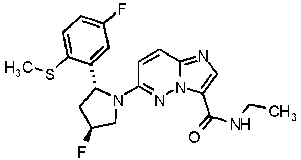 | 6-[(2R,4S)-4-fluoro-2-[5-fluoro-2-(methylsulfanyl)phenyl]pyrrolidin -1-yl]imidazo[1,2-b]pyridazine-3-carboxylic acid (Preparation 17) and ethylamine | Colourles s solid, 77% |
| | N-ethyl-6-[(2R,4S)-4-fluoro-2-[5-fluoro-2-(methylsulfanyl)phenyl]pyrrolidin-1-yl]imidazo[1,2-b]pyridazine-3-carboxamide | | LCMS m/z = 418.0 [M+H]⁺ |
| 23 | 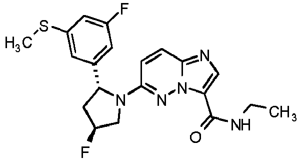 | 6-[(2R,4S)-4-fluoro-2-[5-fluoro-3-(methylsulfanyl)phenyl]pyrrolidin -1-yl]imidazo[1,2-b]pyridazine-3-carboxylic acid (Preparation 18) and ethylamine | Yellow oil, 97% |
| | N-ethyl-6-[(2R,4S)-4-fluoro-2-[5-fluoro-3-(methylsulfanyl)phenyl]pyrrolidin-1-yl]imidazo[1,2-b]pyridazine-3-carboxamide | | LCMS m/z = 418.0 [M+H]⁺ |
| 24 | 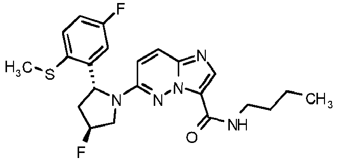 | 6-[(2R,4S)-4-fluoro-2-[5-fluoro-2-(methylsulfanyl)phenyl]pyrrolidin -1-yl]imidazo[1,2-b]pyridazine-3-carboxylic acid (Preparation 17) and n-butylamine | Off-white solid, 92% |
| | N-butyl-6-[(2R,4S)-4-fluoro-2-[5-fluoro-2-(methylsulfanyl)phenyl]pyrrolidin-1-yl]imidazo[1,2-b]pyridazine-3-carboxamide | | LCMS m/z = 446 [M+H]⁺ |

### Preparation 25

### N-cyclohexyl-6-[(2R,4S)-4-fluoro-2-[5-fluoro-2-(methylsulfanyl)phenyl]pyrrolidin-1-yl]imidazo[1,2-b]pyridazine-3-carboxamide

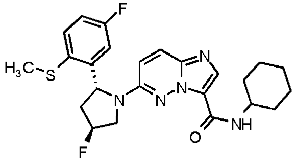

To a solution of 6-[(2R,4S)-4-fluoro-2-[5-fluoro-2-(methylsulfanyl)phenyl]pyrrolidin-1-yl]imidazo[1,2-b]pyridazine-3-carboxylic acid (Preparation 17, 150 mg, 0.380 mmol) in DCM (2 mL) was added cyclohexylamine (46 mg, 0.460 mmol), TBTU (136 mg, 0.460 mmol) and DIPEA (0.132 mL, 0.760 mmol) and the reaction stirred at rt for 1 hr. The mixture was diluted with DCM (10 mL) washed with saturated NH₄Cl soln. (10 mL), dried (MgSO₄)and concentrated *in vacuo* to afford the title compound as a yellow gum, 133 mg, 72%.

LCMS m/z = 472 [M+H]⁺

### Preparation 26

### N-({3-[(tert-butyldimethylsilyl)oxy]phenyl}methyl)-6-[(2R)-2-[5-fluoro-2-(methylsulfanyl)phenyl]pyrrolidin-1-yl]imidazo[1,2-b]pyridazine-3-carboxamide

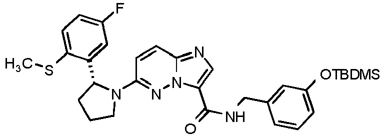

TBDMSCI (59 mg, 0.392 mmol) and 1H-imidazole (44 mg, 0.653 mmol) were added to a solution of 6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboxamide (Preparation 21, 156 mg, 0.327 mmol) in DMF (2 mL) and the reaction stirred at rt for 16 hrs. The mixture was partitioned between MTBE (50 mL) and water (50 mL) and the organic layer washed with saturated brine (3 x 15 mL), dried (Na₂SO₄) and concentrated *in vacuo.* The crude product was purified by column chromatography on silica gel eluting with DCM:MeOH, (99:1 to 92:8) to afford the title compound as a colourless gum, 166 mg, 86%.

LCMS m/z = 592.2 [M+H]⁺

### Preparation 27

### 6-[(2R)-2-[5-fluoro-2-(methylsulfanyl)phenyl]pyrrolidin-1-yl]-N-[(3R)-oxan-3-yl]imidazo[1,2-b]pyridazine-3-carbothioamide

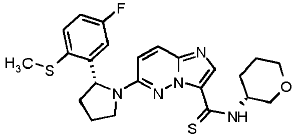

Lawesson's reagent (0.12 g, 0.297 mmol) was added to a solution of 6-[(2R)-2-[5-fluoro-2-(methylsulfanyl)phenyl]pyrrolidin-1-yl]-N-[(3R)-oxan-3-yl]imidazo[1,2-b]pyridazine-3-carboxamide (Preparation 19, 0.113 g, 0.248 mmol) in toluene (2 mL) and the reaction stirred at 100 °C for 16 hrs then cooled to rt. The mixture was concentrated *in vacuo* and the residue purified by column chromatography on silica gel eluting with DCM:MeOH (99:1 to 92:8) to afford the title compound as a yellow solid, 106 mg, 90%.

LCMS m/z = 472 [M+H]⁺

### Preparations 28 to 33

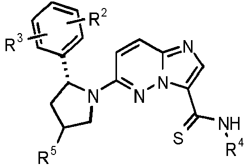

The following compounds were prepared from the appropriate amide and Lawesson's reagent, following the procedure described in Preparation 27.

| Preparation No | Structure and Name | Starting Material | Yield, Data |
|---|---|---|---|
| 28 | 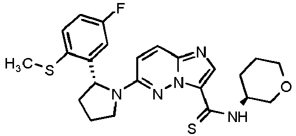 | 6-[(2R)-2-[5-fluoro-2-(methylsulfanyl)phenyl]pyr rolidin-1-yl]-N-[(3S)-oxan-3-yl]imidazo[1,2-b]pyridazine-3-carboxamide (Preparation 20) | Yellow solid, 67% |
| | 6-[(2R)-2-[5-fluoro-2-(methylsulfanyl)phenyl]pyrrolidin-1-yl]-N-[(3S)-oxan-3-yl]imidazo[1,2-b]pyridazine-3-carbothioamide | | LCMS m/z = 472 [M+H]⁺ |
| 29 | 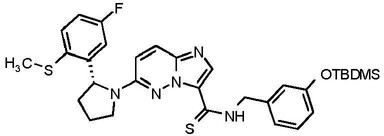 | N-({3-[(tertbutyldimethylsilyl)oxy]phen yl}methyl)-6-[(2R)-2-[5-fluoro-2-(methylsulfanyl)phenyl]pyr rolidin-1-yl]imidazo[1,2-b]pyridazine-3-carboxamide (Preparation 26) | Yellow solid, 67% |
| | N-({3-[(tertbutyldimethylsilyl)oxy]phenyl}methyl)-6-[(2R)-2-[5-fluoro-2-(methylsulfanyl)phenyl]pyrrolidin-1-yl]imidazo[1,2-b]pyridazine-3-carbothioamide | | LCMS m/z = 608.2 [M+H]⁺ |
| 30 | 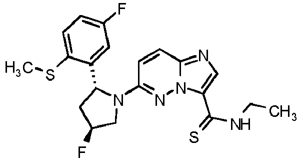 | N-ethyl-6-[(2R,4S)-4-fluoro-2-[5-fluoro-2-(methylsulfanyl)phenyl]pyr rolidin-1-yl]imidazo[1,2-b]pyridazine-3-carboxamide (Preparation 22) | Yellow solid, 65% |
| | N-ethyl-6-[(2R,4S)-4-fluoro-2-[5-fluoro-2-(methylsulfanyl)phenyl]pyrrolidin-1-yl]imidazo[1,2-b]pyridazine-3-carbothioamide | | LCMS m/z = 434.1 [M+H]⁺ |
| 31 | 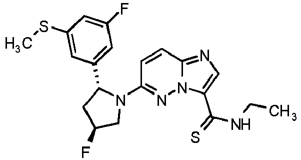 | N-ethyl-6-[(2R,4S)-4-fluoro-2-[5-fluoro-3-(methylsulfanyl)phenyl]pyr rolidin-1-yl]imidazo[1,2-b]pyridazine-3-carboxamide (Preparation 23) | Orange oil, 77% yield. |
| | N-ethyl-6-[(2R,4S)-4-fluoro-2-[5-fluoro-3-(methylsulfanyl)phenyl]pyrrolidin-1-yl]imidazo[1,2-b]pyridazine-3-carbothioamide | | LCMS m/z = 434 [M+H]⁺ |
| 32 | 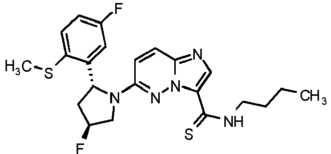 | N-butyl-6-[(2R,4S)-4-fluoro-2-[5-fluoro-2-(methylsulfanyl)phenyl]pyr rolidin-1-yl]imidazo[1,2-b]pyridazine-3-carboxamide (Preparation 24) | Yellow solid, 95% yield. |
| | N-butyl-6-[(2R,4S)-4-fluoro-2-[5-fluoro-2-(methylsulfanyl)phenyl]pyrrolidin-1-yl]imidazo[1,2-b]pyridazine-3-carbothioamide | | LCMS m/z = 462 [M+H]⁺ |
| 33 | 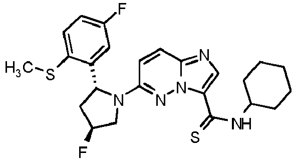 | N-cyclohexyl-6-[(2R,4S)-4-fluoro-2-[5-fluoro-2-(methylsulfanyl)phenyl]pyr rolidin-1-yl]imidazo[1,2-b]pyridazine-3-carboxamide (Preparation 25) | Yellow solid, quantitativ e yield |
| | N-cyclohexyl-6-[(2R,4S)-4-fluoro-2-[5-fluoro-2-(methylsulfanyl)phenyl]pyrrolidin-1-yl]imidazo[1,2-b]pyridazine-3-carbothioamide | | LCMS m/z = 488 [M+H]⁺ |

### Preparation 34

### (Z)-N-({3-[(tert-butyldimethylsilyl)methyl]phenyl}methyl)-N'-cyano-6-[(2R)-2-[5-fluoro-2-(methylsulfanyl)phenyl]pyrrolidin-1-yl]imidazo[1,2-b]pyridazine-3-carboximidamide

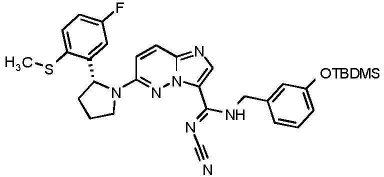

Mercury dichloride (111 mg, 0.411 mmol) followed by cyanamide (50 mg, 0.493 mmol) were added to a solution of N-({3-[(tert-butyldimethylsilyl)oxy]phenyl}methyl)-6-[(2R)-2-[5-fluoro-2-(methylsulfanyl)phenyl]pyrrolidin-1-yl]imidazo[1,2-b]pyridazine-3-carbothioamide (Preparation 29, 100 mg, 0.164 mmol) in DMF (2 mL) under N₂(g) and the reaction stirred at rt for 16 hrs. The mixture was concentrated *in vacuo,* re-suspended in DCM (10 mL), filtered through a pad of Dicalite^{®}, rinsing through with 20% MeOH in DCM (20 mL). The filtrate was concentrated *in vacuo* and purified by column chromatography on silica gel eluting with heptanes:EtOAc:MeOH, (80:20:0 to 0:100:0 to 0:90:10) to afford the title compound as a colourless gum, 60 mg, 59%.

LCMS m/z = 616 [M+H]⁺

## Claims

1. A compound of Formula (I): 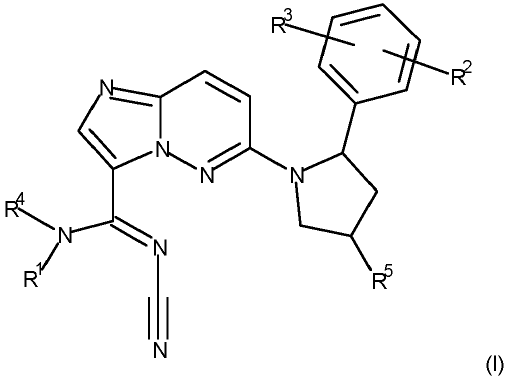 or a pharmaceutically acceptable salt or solvate thereof;
wherein:
R¹ is selected from H, -XR⁷, (C₁-C₆)alkyl, (C₃-C₈)cycloalkyl, and a C-linked 4-6 membered heterocycloalkyl containing 1 to 2 heteroatoms selected from N, O and S;
X is selected from -CH₂-;
R² is selected from H and -SR⁶;
R³ is selected from H and halo;
R⁴ is selected from H and (C₁-C₃)alkyl
R⁵ is selected from H and halo;
R⁶ is methyl;
R⁷ is phenyl substituted by hydroxy wherein the hydroxyphenyl is optionally further substituted by halo;
provided that if R² is H then R¹ is XR⁷.

2. A compound according to claim 1 wherein R¹ is selected from -XR⁷, (C₁-C₆)alkyl, (C₃-C₈)cycloalkyl, and a C-linked 4-6 membered heterocycloalkyl containing 1 to 2 heteroatoms selected from N, O and S.

3. A compound according to claim 1 or 2 wherein R¹ is selected from (C₁-C₆)alkyl and (C₃-C₈)cycloalkyl.

4. A compound according to claim 3 wherein R¹ is (C₁-C₆)alkyl.

5. A compound according to claim 1 or 2 wherein R¹ is selected from -XR⁷ and a C-linked 4-6 membered heterocycloalkyl containing 1 to 2 heteroatoms selected from N, O and S.

6. A compound according to claim 5 wherein R¹ is selected from -XR⁷ and a C-linked 4-6 membered heterocycloalkyl containing 1 to 2 heteroatoms selected from N and O.

7. A compound according to any preceding claim wherein
(i) R² is -SR⁶;
(ii) R³ is H or fluoro;
(iii) R⁴ is H; and/or
(iv) R⁵ is H or fluoro.

8. A compound according to any preceding claim wherein R⁷ is phenyl substituted by hydroxy wherein the hydroxyphenyl is optionally further substituted by fluoro.

9. A compound of Formula la: 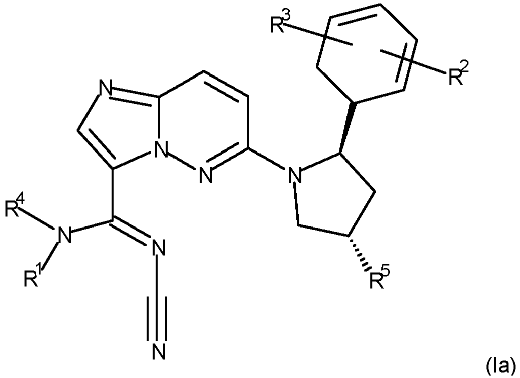 or a pharmaceutically salt or solvate thereof, wherein R¹, R², R³, R⁴and R⁵ are as defined in any preceding claim.

10. A compound according to claim 1, wherein said compound is selected from:
N'-cyano-6-[2-[5-fluoro-2-(methylsulfanyl)phenyl]pyrrolidin-1-yl]-N-[(3R)-oxan-3-yl]imidazo[1,2-b]pyridazine-3-carboximidamide;
N'-cyano-6-[2-[5-fluoro-2-(methylsulfanyl)phenyl]pyrrolidin-1-yl]-N-[(oxan-3-yl]imidazo[1,2-b]pyridazine-3-carboximidamide;
N'-cyano-N-ethyl-6-[4-fluoro-2-[5-fluoro-3-(methylsulfanyl)phenyl]pyrrolidin-1-yl]imidazo[1,2-b]pyridazine-3-carboximidamide;
N'-cyano-N-ethyl-6-[4-fluoro-2-[5-fluoro-2-(methylsulfanyl)phenyl]pyrrolidin-1-yl]imidazo[1,2-b]pyridazine-3-carboximidamide;
N-butyl-N'-cyano-6-[4-fluoro-2-[5-fluoro-2-(methylsulfanyl)phenyl]pyrrolidin-1-yl]imidazo[1,2-b]pyridazine-3-carboximidamide;
N'-cyano-N-cyclohexyl-6-[4-fluoro-2-[5-fluoro-2-(methylsulfanyl)phenyl]pyrrolidin-1-yl]imidazo[1,2-b]pyridazine-3-carboximidamide; and
N'-cyano-6-[2-[5-fluoro-2-(methylsulfanyl)phenyl]pyrrolidin-1-yl]-N-[(3-hydroxyphenyl)methyl]imidazo[1,2-b]pyridazine-3-carboximidamide;
or a pharmaceutically acceptable salt or solvate thereof.

11. A compound according to claim 1 or claim 9, wherein said compound is selected from:
(Z)-N'-cyano-6-[(2R)-2-[5-fluoro-2-(methylsulfanyl)phenyl]pyrrolidin-1-yl]-N-[(3R)-oxan-3-yl]imidazo[1,2-b]pyridazine-3-carboximidamide ;
(Z)-N'-cyano-6-[(2R)-2-[5-fluoro-2-(methylsulfanyl)phenyl]pyrrolidin-1-yl]-N-[(3S)-oxan-3-yl]imidazo[1,2-b]pyridazine-3-carboximidamide;
(Z)-N'-cyano-N-ethyl-6-[(2R,4S)-4-fluoro-2-[5-fluoro-3-(methylsulfanyl)phenyl]pyrrolidin-1-yl]imidazo[1,2-b]pyridazine-3-carboximidamide;
(Z)-N'-cyano-N-ethyl-6-[(2R,4S)-4-fluoro-2-[5-fluoro-2-(methylsulfanyl)phenyl]pyrrolidin-1-yl]imidazo[1,2-b]pyridazine-3-carboximidamide;
(Z)-N-butyl-N'-cyano-6-[(2R,4S)-4-fluoro-2-[5-fluoro-2-(methylsulfanyl)phenyl]pyrrolidin-1-yl]imidazo[1,2-b]pyridazine-3-carboximidamide;
(Z)-N'-cyano-N-cyclohexyl-6-[(2R,4S)-4-fluoro-2-[5-fluoro-2-(methylsulfanyl)phenyl]pyrrolidin-1-yl]imidazo[1,2-b]pyridazine-3-carboximidamide; and
(Z)-N'-cyano-6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboximidamide;
or a pharmaceutically acceptable salt or solvate thereof.

12. A pharmaceutical composition comprising a compound according to any of claims 1 to 11 and one or more pharmaceutically acceptable excipients.

13. A pharmaceutical composition according to claim 12 in combination with one or more other therapeutic agents.

14. A compound according to any of claims 1 to 11 for use as a pharmaceutical.

15. A compound according to any of claims 1 to 11 for use in the treatment of atopic dermatitis.

## Patentansprüche

1. Verbindung der Formel (I): 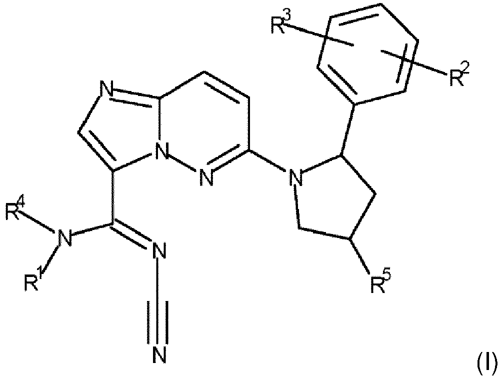 oder ein pharmazeutisch verträgliches Salz oder Solvat davon;
wobei:
R¹ ausgewählt ist aus H, -XR⁷, (C₁-C₆)-Alkyl, (C₃-C₈)-Cycloalkyl und einem C-verknüpften 4-6-gliedrigen Heterocycloalkyl, das 1 bis 2 Heteroatome enthält, die aus N, O und S ausgewählt sind;
X aus -CH₂- ausgewählt ist;
R² aus H und -SR⁶ ausgewählt ist;
R³ aus H und Halogen ausgewählt ist;
R⁴ aus H und (C₁-C₃)-Alkyl ausgewählt ist
R⁵ aus H und Halogen ausgewählt ist;
R⁶ Methyl ist;
R⁷ Phenyl ist, das durch Hydroxy substituiert ist, wobei das Hydroxyphenyl optional ferner durch Halogen substituiert ist;
vorausgesetzt, dass, falls R² H ist, dann R¹ XR⁷ ist.

2. Verbindung nach Anspruch 1, wobei R¹ ausgewählt ist aus -XR⁷, (C₁-C₆)-Alkyl, (C₃-C₈)-Cycloalkyl und einem C-verknüpften 4-6-gliedrigen Heterocycloalkyl, das 1 bis 2 Heteroatome enthält, die aus N, O und S ausgewählt sind.

3. Verbindung nach Anspruch 1 oder 2, wobei R¹ ausgewählt ist aus (C₁-C₆)-Alkyl, (C₃-C₈)-Cycloalkyl.

4. Verbindung nach Anspruch 3, wobei R¹ (C₁-C₆)-Alkyl ist.

5. Verbindung nach Anspruch 1 oder 2, wobei R¹ ausgewählt ist aus -XR⁷ und einem C-verknüpften 4-6-gliedrigen Heterocycloalkyl, das 1 bis 2 Heteroatome enthält, die aus N, O und S ausgewählt sind.

6. Verbindung nach Anspruch 5, wobei R¹ ausgewählt ist aus -XR⁷ und einem C-verknüpften 4-6-gliedrigen Heterocycloalkyl, das 1 bis 2 Heteroatome enthält, die aus N und O ausgewählt sind.

7. Verbindung nach einem der vorstehenden Ansprüche, wobei
(i) R² -SR⁶ ist;
(ii) R³ H oder Fluor ist;
(iii) R⁴ H ist; und/oder
(iv) R⁵ H oder Fluor ist.

8. Verbindung nach einem der vorstehenden Ansprüche, wobei R⁷ Phenyl ist, das durch Hydroxy substituiert ist, wobei das Hydroxyphenyl optional ferner durch Fluor substituiert ist.

9. Verbindung der Formel Ia: 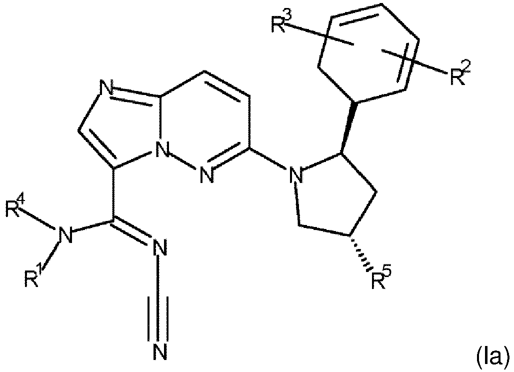 oder ein pharmazeutisches Salz oder Solvat davon, wobei R¹, R², R³, R⁴ und R⁵ wie in einem der vorstehenden Ansprüche definiert sind.

10. Verbindung nach Anspruch 1, wobei die Verbindung ausgewählt ist aus:
N'-cyano-6-[2-[5-fluoro-2-(methylsulfanyl)phenyl]pyrrolidin-1-yl]-N-[(3R)-oxan-3-yl]imidazo[1,2-b]pyridazine-3-carboximidamid;
N'-cyano-6-[2-[5-fluoro-2-(methylsulfanyl)phenyl]pyrrolidin-1-yl]-N-[(oxan-3-yl]imidazo[1,2-b]pyridazine-3-carboximidamid;
N'-cyano-N-ethyl-6-[4-fluoro-2-[5-fluoro-3-(methylsulfanyl)phenyl]pyrrolidin-1-yl]imidazo[1,2-b]pyridazine-3-carboximidamid;
N'-cyano-N-ethyl-6-[4-fluoro-2-[5-fluoro-2-(methylsulfanyl)phenyl]pyrrolidin-1-yl]imidazo[1,2-b]pyridazine-3-carboximidamid;
N-butyl-N'-cyano-6-[4-fluoro-2-[5-fluoro-2-(methylsulfanyl)phenyl]pyrrolidin-1-yl]imidazo[1,2-b]pyridazine-3-carboximidamid;
N'-cyano-N-cyclohexyl-6-[4-fluoro-2-[5-fluoro-2-(methylsulfanyl)phenyl]pyrrolidin-1-yl]imidazo[1,2-b]pyridazine-3-carboximidamid; und
N'-cyano-6-[2-[5-fluoro-2-(methylsulfanyl)phenyl]pyrrolidin-1-yl]-N-[(3-hydroxyphenyl)methyl]imidazo[1,2-b]pyridazine-3-carboximidamid;
oder einem pharmazeutisch verträglichen Salz oder Solvat davon.

11. Verbindung nach Anspruch 1 oder 9, wobei die Verbindung ausgewählt ist aus:
(Z)-N'-cyano-6-[(2R)-2-[5-fluoro-2-(methylsulfanyl)phenyl]pyrrolidin-1-yl]-N-[(3R)-oxan-3-yl]imidazo[1,2-b]pyridazine-3-carboximidamid;
(Z)-N'-cyano-6-[(2R)-2-[5-fluoro-2-(methylsulfanyl)phenyl]pyrrolidin-1-yl]-N-[(3S)-oxan-3-yl]imidazo[1,2-b]pyridazine-3-carboximidamid;
(Z)-N'-cyano-N-ethyl-6-[(2R,4S)-4-fluoro-2-[5-fluoro-3-(methylsulfanyl)phenyl]pyrrolidin-1-yl]imidazo[1,2-b]pyridazine-3-carboximidamid;
(Z)-N'-cyano-N-ethyl-6-[(2R,4S)-4-fluoro-2-[5-fluoro-2-(methylsulfanyl)phenyl]pyrrolidin-1-yl]imidazo[1,2-b]pyridazine-3-carboximidamid;
(Z)-N-butyl-N'-cyano-6-[(2R,4S)-4-fluoro-2-[5-fluoro-2-(methylsulfanyl)phenyl]pyrrolidin-1-yl]imidazo[1,2-b]pyridazine-3-carboximidamid;
(Z)-N'-cyano-N-cyclohexyl-6-[(2R,4S)-4-fluoro-2-[5-fluoro-2-(methylsulfanyl)phenyl]pyrrolidin-1 -yl]imidazo[1,2-b]pyridazine-3-carboximidamid;
und (Z)-N'-cyano-6-[(2R)-2-[5-fluoro-2-(methylsulfanyl)phenyl]pyrrolidin-1-yl]-N-[(3-hydroxyphenyl)methyl]imidazo[1,2-b]pyridazine-3-carboximidamid;
oder einem pharmazeutisch verträglichen Salz oder Solvat davon.

12. Pharmazeutische Zusammensetzung, umfassend eine Verbindung nach einem der Ansprüche 1 bis 11 und einen oder mehrere pharmazeutisch verträgliche Hilfsstoffe.

13. Pharmazeutische Zusammensetzung nach Anspruch 12 in Kombination mit einem oder mehreren anderen therapeutischen Wirkstoffen.

14. Verbindung nach einem der Ansprüche 1 bis 11 zur Verwendung als ein Arzneimittel.

15. Verbindung nach einem der Ansprüche 1 bis 11 zur Verwendung bei der Behandlung von atopischer Dermatitis.

## Revendications

1. Composé de Formule (I) : 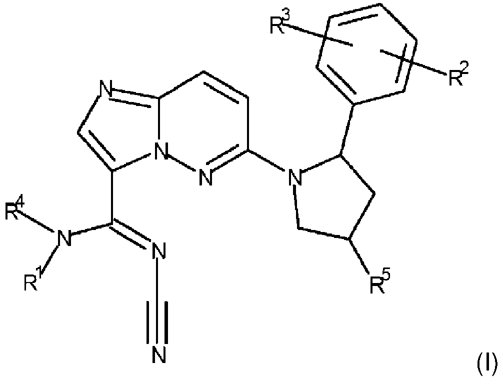 ou un sel ou solvate pharmaceutiquement acceptable de celui-ci ;
dans lequel :
R¹ est choisi parmi H, -XR⁷, alkyle en C₁-C₆, alkyle en C₃-C₈ et un hétérocycloalkyle à 4 à 6 chaînons à liaison C contenant 1 à 2 hétéroatomes choisis parmi N, O et S ;
X est choisi parmi -CH₂- ;
R² est choisi parmi H et -SR⁶ ;
R³ est choisi parmi H et halogéno ;
R⁴ est choisi parmi H et alkyle en C₁-C₃
R⁵ est choisi parmi H et halogéno ;
R⁶ est méthyle ;
R⁷ est phényle substitué par hydroxy, l'hydroxyphényle étant facultativement substitué en outre par halogéno ;
à condition que si R² est H, alors R¹ est XR⁷.

2. Composé selon la revendication 1, dans lequel R¹ est choisi parmi -XR⁷, alkyle en C₁-C₆, cycloalkyle en C₃-C₈ et un hétérocycloalkyle à 4 à 6 chaînons à liaison C contenant 1 à 2 hétéroatomes choisis parmi N, O et S.

3. Composé selon la revendication 1 ou 2, dans lequel R¹ est choisi parmi alkyle en C₁-C₆ et cycloalkyle en C₃-C₈.

4. Composé selon la revendication 3, dans lequel R¹ est alkyle en C₁-C₆.

5. Composé selon la revendication 1 ou 2, dans lequel R¹ est choisi parmi - XR⁷ et un hétérocycloalkyle à 4 à 6 chaînons à liaison C contenant 1 à 2 hétéroatomes choisis parmi N, O et S.

6. Composé selon la revendication 5, dans lequel R¹ est choisi parmi -XR⁷ et un hétérocycloalkyle à 4 à 6 chaînons à liaison C contenant 1 à 2 hétéroatomes choisis parmi N et O.

7. Composé selon une quelconque revendication précédente, dans lequel
(i) R² est -SR⁶ ;
(ii) R³ est H ou fluoro ;
(iii) R⁴ est H ; et/ou
(iv) R⁵ est H ou fluoro.

8. Composé selon une quelconque revendication précédente, dans lequel R⁷ est phényle substitué par hydroxy, l'hydroxyphényle étant facultativement substitué en outre par fluoro.

9. Composé de Formule la : 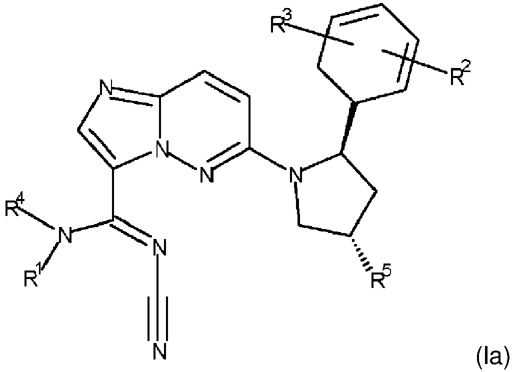 ou un sel ou solvate pharmaceutiquement acceptable de celui-ci, dans lequel R¹ R², R³, R⁴ et R⁵ sont tels que définis dans une quelconque revendication précédente.

10. Composé selon la revendication 1, dans lequel ledit composé est choisi parmi :
N'-cyano-6-[2-[5-fluoro-2-(méthylsulfanyl)phényl]pyrrolidin-1-yl]-N-[(3R)-oxan-3-yl]imidazo[1,2-b]pyridazine-3-carboximidamide ;
N'-cyano-6-[2-[5-fluoro-2-(méthylsulfanyl)phényl]pyrrolidin-1-yl]-N-[(oxan-3-yl]imidazo[1,2-b]pyridazine-3-carboximidamide ;
N'-cyano-N-éthyl-6-[4-fluoro-2-[5-fluoro-3-(méthylsulfanyl)phényl]pyrrolidin-1-yl]imidazo[1,2-b]pyridazine-3-carboximidamide ;
N'-cyano-N-éthyl-6-[4-fluoro-2-[5-fluoro-2-(méthylsulfanyl)phényl]pyrrolidin-1-yl]imidazo[1,2-b]pyridazine-3-carboximidamide ;
N-butyl-N'-cyano-6-[4-fluoro-2-[5-fluoro-2-(méthylsulfanyl)phényl]pyrrolidin-1-yl]imidazo[1,2-b]pyridazine-3-carboximidamide ;
N'-cyano-N-cyclohexyl-6-[4-fluoro-2-[5-fluoro-2-(méthylsulfanyl)phényl]pyrrolidin-1-yl]imidazo[1,2-b]pyridazine-3-carboximidamide ; et
N'-cyano-6-[2-[5-fluoro-2-(méthylsulfanyl)phényl]pyrrolidin-1-yl]-N-[(3-hydroxyphényl)méthyl]imidazo[1,2-b]pyridazine-3-carboximidamide ;
ou un sel ou solvate pharmaceutiquement acceptable de celui-ci.

11. Composé selon la revendication 1 ou la revendication 9, dans lequel ledit composé est choisi parmi :
(Z)-N'-cyano-6-[(2R)-2-[5-fluoro-2-(méthylsulfanyl)phényl]pyrrolidin-1-yl]-N-[(3R)-oxan-3-yl]imidazo[1,2-b]pyridazine-3-carboximidamide ;
(Z)-N'-cyano-6-[(2R)-2-[5-fluoro-2-(méthylsulfanyl)phényl]pyrrolidin-1-yl]-N-[(3S)-oxan-3-yl]imidazo[1,2-b]pyridazine-3-carboximidamide ;
(Z)-N'-cyano-N-éthyl-6-[(2R,4S)-4-fluoro-2-[5-fluoro-3-(méthylsulfanyl)phényl]pyrrolidin-1-yl]imidazo[1,2-b]pyridazine-3-carboximidamide ;
(Z)-N'-cyano-N-éthyl-6-[(2R,4S)-4-fluoro-2-[5-fluoro-2-(méthylsulfanyl)phényl]pyrrolidin-1-yl]imidazo[1,2-b]pyridazine-3-carboximidamide ;
(Z)-N-butyl-N'-cyano-6-[(2R,4S)-4-fluoro-2-[5-fluoro-2-(méthylsulfanyl)phényl]pyrrolidin-1-yl]imidazo[1,2-b]pyridazine-3-carboximidamide ;
(Z)-N'-cyano-N-cyclohexyl-6-[(2R,4S)-4-fluoro-2-[5-fluoro-2-(méthylsulfanyl)phényl]pyrrolidin-1-yl]imidazo[1,2-b]pyridazine-3-carboximidamide ; et (Z)-N'-cyano-6-[(2R)-2-[5-fluoro-2-(méthylsulfanyl)phényl]pyrrolidin-1-yl]-N-[(3-hydroxyphényl)méthyl]imidazo[1,2-b]pyridazine-3-carboximidamide ;
ou un sel ou solvate pharmaceutiquement acceptable de celui-ci.

12. Composition pharmaceutique comprenant un composé selon l'une quelconque des revendications 1 à 11 et un ou plusieurs excipients pharmaceutiquement acceptables.

13. Composition pharmaceutique selon la revendication 12 en combinaison avec un ou plusieurs autres agents thérapeutiques.

14. Composé selon l'une quelconque des revendications 1 à 11 destiné à être utilisé en tant que produit pharmaceutique.

15. Composé selon l'une quelconque des revendications 1 à 11 destiné à être utilisé dans le traitement de la dermatite atopique.
